# EUROPEAN PATENT SPECIFICATION

(11) **EP 2 890 376 B1**
(45) Date of publication and mention of the grant of the patent: **27.09.2017**
(21) Application number: 12883653.3
(22) Date of filing: 15.10.2012
(51) Int. Cl.: A61K 31/496, A61P 7/02, A61P 7/00

(54) **METHODS AND COMPOSITIONS FOR HYPOTENSIVE RESUSCITATION**
VERFAHREN UND ZUSAMMENSETZUNGEN ZUR WIEDERBELEBUNG HYPOTENSIVER PATIENTEN
PROCÉDÉ ET COMPOSITIONS POUR LA RÉANIMATION HYPOTENSIVE

(30) Priority: 31.08.2012 IN CH36172012
(43) Date of publication of application: 08.07.2015
(73) Proprietor: Pharmazz, Inc., Willowbrook, IL 60527 (US); Midwestern University, Downers Grove, IL 60515 (US)
(72) Inventor: GULATI, Anil, Naperville, IL 60565 (US); LAVHALE, Manish, S., Amravati 444605 (IN); ANDURKAR, Shridhar, V., Naperville, IL 60565 (US)
(74) Representative: Studio Torta S.p.A.
(86) International application number: PCT/US2012/060257
(87) International publication number: WO 2014/035446

(56) References cited:
- US-A- 3 983 121
- US-A1- 2012 083 447
- US-B1- 6 369 114
- ANIL GULATI ET AL: "Centhaquin improves resuscitative effect of hypertonic saline in hemorrhaged rats", JOURNAL OF SURGICAL RESEARCH., vol. 178, no. 1, 2 April 2012 (2012-04-02) , pages 415-423, XP055232717, US ISSN: 0022-4804, DOI: 10.1016/j.jss.2012.02.005
- GULATI, A. ET AL.: 'Centhaquin improves resuscitative effect of hypertonic saline in hemorrhaged rats.' JOURNAL OF SURGICAL RESEARCH vol. 178, November 2012, pages 415 - 423, XP055232717
- BAJPAI, U. C. ET AL.: 'Fourier transform infrared spectra and normal mode analysis of 1-(3-methyl phenyl piperazin-1-yl)-2-(quinolin-2-yl)ethane (Centhaquin): a potent centrally acting anti-hypertensive agent' JOURNAL OF MOLECULAR STRUCTURE vol. 516, no. 1, 04 January 2000, pages 15 - 21, XP055232721

## Description

### FIELD OF THE INVENTION

The present invention is directed to methods and compositions for reversing end points of shock by improving cardiac output, administering the α₂ adrenergic agent centhaquin citrate to a mammal, including humans. The α₂ adrenergic agent centhaquin citrate is administered at a low dose, and typically in conjunction with a low volume of a resuscitation fluid, like a colloid solution, a crystalloid solution, blood, or a blood component. The present invention also is directed to purified centhaquin and centhaquin citrate, and their methods of preparation.

### BACKGROUND OF THE INVENTION

Shock due to severe hemorrhage accounts for a large proportion of posttraumatic deaths, particularly during early stages of injury (Wu, Dai et al. 2009). A majority of deaths due to hemorrhage occur within the first six hours after trauma (Shackford, Mackersie et al. 1993), but many of these deaths can be prevented (Acosta, Yang et al. 1998).

Shock is accompanied by circulatory failure which is the primary cause of mortality and morbidity. Presently, the recommended fluid therapy uses large volumes of Lactated Ringer's solution (LR), which is effective in restoring hemodynamic parameters, but presents logistic and physiologic limitations (Vincenzi, Cepeda et al. 2009). For example, resuscitation using a large volume of crystalloids, like LR, has been associated with secondary abdominal compartment syndrome, pulmonary edema, cardiac dysfunction, and paralytic ileus (Balogh, McKinley et al. 2003). Therefore, a need exists in the art for a resuscitation agent that improves survival time, and can be used with a small volume of resuscitation fluid, for resuscitation in hypovolemic shock.

Centhaquin (2-[2-(4-(3-methyphenyl)-1-piperazinyl) ethyl-quinoline) is a centrally acting antihypertensive drug. The structure of centhaquin was determined (Bajpai et al., 2000) and the conformation of centhaquin was confirmed by X-ray diffraction (Carpy and Saxena, 1991).

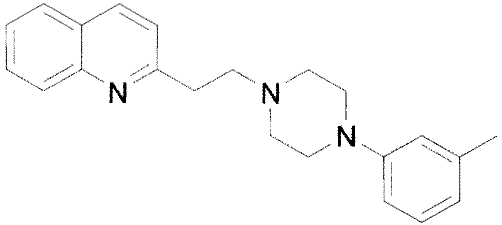

### Structure ofcenthaquin (2-[2-(4-(3-methyphenyl)-1-piperazinyl) ethyl]-quinoline) (as free base)

Centhaquin is an active cardiovascular agent that produces a positive inotropic effect and increases ventricular contractions of isolated perfused rabbit heart (Bhatnagar, Pande et al. 1985). Centhaquin does not affect spontaneous contractions of the guinea pig right auricle, but significantly potentiates positive inotropic effect of norepinephrine (NE) (Srimal, Mason et al. 1990). The direct or indirect positive inotropic effect of centhaquin can lead to an increase in cardiac output (CO). Centhaquin produces a decrease in mean arterial pressure (MAP) and heart rate (HR) in anesthetized rats and conscious freely moving cats and rats (Srimal, Gulati et al. 1990) due to its central sympatholytic activity (Murti, Bhandari et al. 1989; Srimal, Gulati et al. 1990; Gulati, Hussain et al. 1991). When administered locally into a dog femoral artery, centhaquin (10 and 20 µg) increased blood flow, which was similar to that observed with acetylcholine and papaverine. However, the vasodilator effect of centhaquin could not be blocked by atropine or dibenamine (Srimal, Mason et al. 1990). The direct vasodilator or central sympatholytic effect of centhaquin is likely to decrease systemic vascular resistance (SVR).

It was found that centhaquin enhances the resuscitative effect of hypertonic saline (HS) (Gulati, Lavhale et al. 2012). Centhaquin significantly decreased blood lactate and increases MAP, stroke volume, and CO compared to hypertonic saline alone. It is theorized, but not relied upon, that the cardiovascular actions of hypertonic saline and centhaquin are mediated through the ventrolateral medulla in the brain (Gulati, Hussain et al. 1991; Cavun and Millington 2001) and centhaquin may be augmenting the effect of hypertonic saline.

A large volume of LR (i.e., about three times the volume of blood loss) is the most commonly used resuscitation fluid therapy (Chappell, Jacob et al. 2008), in part because LR does not exhibit the centrally mediated cardiovascular effects of hypertonic saline. Large volume resuscitation has been used by emergency medical personnel and surgeons to reverse hemorrhagic shock and to restore end-organ perfusion and tissue oxygenation. However, there has been a vigorous debate with respect to the optimal methods of resuscitation (Santry and Alam 2010). Various documents like US2012083447, Gulati et al. (2011 and 2012) discuss the rescucitative effects in shock of centhaquin (free base). Bajpai et al (2000) generally refers to centhaquin and analogs thereof as potent centrally acting anti-hypertnesive agents. US3983121 discloses the synthesis of pharmacologically active substances, viz. centhaquin and analogues, i.a. centhaquin citrate.

### SUMMARY OF THE INVENTION

The present invention is directed to compositions for use in treating hypotensive shock in mammals, including humans. More specifically, the present invention provides for the administration of the α₂ adrenergic agent, centhaquin citrate in a therapeutically effective dose to reverse important end points of shock and act as an effective resuscitation agent.

Further disclosed herein are methods of treating diseases and conditions wherein an increased cardiac output, increased tissue oxygenation, and increased oxygen perfusion provides a benefit comprising administering a low dose of the
α₂ adrenergic centhaquine citrate, alone or with a resuscitation fluid, such as a colloid solution, a crystalloid solution, blood, or a blood component.

In yet another embodiment, the α₂ adrenergic agent centhaquin citrate is administered with a resuscitation fluid administered in a volume amount of up to three times the volume amount of body fluid, e.g., blood, plasma, electrolytes, or water. More preferably, the resuscitation fluid is administered in a volume amount less than and up to the volume amount of lost body fluid.

In another embodiment, the cardiac output, tissue oxygenation, or organ perfusion in a mammal is improved by the administration of centhaquin citrate.

It is further disclosed herein a method to provide a purified centhaquin and a purified centhaquin citrate, and methods of manufacturing the same.

Yet another aspect of the present invention is to provide an article of manufacture for human pharmaceutical use comprising (a) a package insert, (b) a container, and (c) a packaged composition comprising the α₂ adrenergic agent centhaquin citrate, and optionally, a packaged resuscitation fluid. The package insert includes instructions for the use of centhaquin citrate in improving cardiac output, in an individual suffering from shock according to claim 1, such as hypovolemic shock, dengue shock syndrome, and septic shock.

These and other aspects and embodiments of the present invention will become apparent from the following detailed description of the invention.

### BRIEF DESCRIPTION OF THE DRAWINGS

Fig. 1(a) and (b) are high resolution mass spectra of centhaquin and centhaquin citrate, respectively.
Figure 2 contains plots showing the cardiovascular effects of centhaquin (0.05, 0.15 and 0.45 mg/kg) and centhaquin citrate dihydrate (0.05,0.15 and 0.45 mg/kg) on mean arterial pressure in urethane anaesthetized rats.
Figure 3 contains plots showing the cardiovascular effects of centhaquin (0.05, 0.15 and 0.45 mg/kg) and centhaquin citrate dihydrate (0.05, 0.15 and 0.45 mg/kg) on pulse pressure in urethane anaesthetized rats.
Figure 4 contains plots showing the cardiovascular effects of centhaquin (0.05,0.15 and 0.45 mg/kg) and centhaquin citrate dihydrate (0.05, 0.15 and 0.45 mg/kg) on heart rate in urethane anaesthetized rats.
Figure 5 contains plots showing the cardiovascular effects of centhaquin (0.05, 0.15 and 0.45 mg/kg) and centhaquin citrate dihydrate (0.05, 0.15 and 0.45 mg/kg) on cardiac output in urethane anaesthetized rats.
Figure 6 contains plots showing the cardiovascular effects of centhaquin (0.05,0.15 and 0.45 mg/kg) and centhaquin citrate dihydrate (0.05, 0.15 and 0.45 mg/kg) on stroke volume in urethane anaesthetized rats.
Figure 7 contains plots showing the cardiovascular effects of centhaquin (0.05, 0.15 and 0.45 mg/kg) and centhaquin citrate dihydrate (0.05, 0.15 and 0.45 mg/kg) on stroke work in urethane anaesthetized rats.
Figure 8 contains plots showing the effect of centhaquin on blood hematocrit in hemorrhaged rats.
Figure 9 contains plots showing the effect of centhaquin on blood lactate and base deficit in hemorrhaged rats.
Figure 10 contains plots showing the effect of centhaquin on mean arterial pressure and heart rate in hemorrhaged rats.
Figure 11 contains plots showing the effect of centhaquin on cardiac output and stroke volume in hemorrhaged rats.
Figure 12 contains plots showing the effect of centhaquin on systemic vascular resistance and stroke work in hemorrhaged rats.
Figure 13 is a survival curve for the vehicle and the treatment groups in hemorrhaged rats.
Figure 14 contains plots of mean arterial pressure (mmHg) and heart rate (beats per minute) versus time for rats in endotoxic shock showing the effect Lipopolysaccharde (LPS) administration with vehicle and with centhaquin citrate dihydrate (0.05 mg/kg).

### DETAILED DESCRIPTION OF THE PREFERRED EMBODIMENTS

The present invention is directed to the administration of the α₂ adrenergic agent centhaquin citrate to treat a condition or diseases wherein an improved cardiac output, an improved tissue oxygenation, and improved organ perfusion provides a benefit.

The methods described herein benefit from the use of the α₂ adrenergic centhaquin citrate. The centhaquin citrate can be administered with a resuscitation fluid, like a colloid solution or a crystalloid solution, typically used in the treatment of resuscitative shock.

For the purposes of the invention disclosed herein, the term "treatment" includes lowering, ameliorating, or eliminating the end points of shock and associated symptoms. As such, the term "treatment" includes medical therapeutic administration.

The term "container" means any receptacle and closure therefore suitable for storing, shipping, dispensing, and/or handling a pharmaceutical product.

The term "insert" means information accompanying a product that provides a description of how to administer the product, along with the safety and efficacy data required to allow the physician, pharmacist, and patient to make an informed decision regarding use of the product. The package insert generally is regarded as the "label" for a pharmaceutical product.

The term "α₂ adrenergic agent" means a compound that stimulates the sympathetic nervous system, e.g., that mimics the effects of norepinephrine and epinephrine. As used herein the term "α₂ adrenergic agent" is singular or plural. Nonlimiting examples of α₂ adrenergic agents include, but are not limited to, centhaquin, clonidine, guanfacine, guanabenz, guanoxabenz, guanethidine, xylazine, tizanidine, methyldopa, fadolmidine, amidephrine, amitraz, anisodamine, apraclonidine, brimonidine, cirazoline, detomidine, dexmedetomidine, epinephrine, ergotamine, etilefrine, indanidine, lofexidine, medetomidine, mephentermine, metaraminol, methoxamine, mivazerol, naphazoline, norepinephrine, norfenefrine, octopamine, oxymetazoline, phenylpropanolamine, rilmenidine, romifidine, synephrine, talipexole, salts thereof, and mixtures thereof.

In accordance with the present invention, tests were performed to determine the resuscitative effect of a representative α₂ adrenergic agent, i.e., centhaquin citrate. However, persons skilled in the art recognize that the results presented below for centhaquin citrate also would be demonstrated by other α₂ adrenergic agents.

Because hypertonic saline exhibits cardiovascular effects, the tests were performed using Lactated Ringer's solution (LR), which does not exhibit any centrally mediated cardiovascular effects. This permits the determination of cardiovascular effects attributable to centhaquin or other α₂ adrenergic agent alone. Because a large volume of LR typically is used in fluid therapy, the resuscitative effect of centhaquin was compared to LR administered at three times the volume of fluid loss, termed herein as "LR-300". As also used herein, the term "LR-100" means LR administered at a volume less than and up to equal the volume of fluid loss.

In the studies disclosed below, a rodent model of fixed-pressure hemorrhage without tissue trauma was used. This model limits factors that can influence the activity of a drug that is being investigated for the first time as a resuscitation agent. This hemorrhage model was made more severe by maintaining the hypotension for 30 minutes from the onset of hemorrhage to reach a base deficit of greater than -12 mEq/L (Gulati, Sen et al. 1997). In this irreversible situation, compensatory mechanisms fail (decompensatory phase), and a tremendous decrease in blood flow to all organs occurs, causing hypoperfusion leading to tissue hypoxia and end-organ failure (Gulati, Sen et al. 1997).

It was found that, in control rats, LR-100 was not effective in improving the time of survival, reversing base deficit, decreasing blood lactate levels, or improving MAP or CO. This suggests that there is persistent oxygen debt and inadequate resuscitation with LR-100. On the other hand, centhaquin improved all these parameters and was an effective resuscitation agent. These results support the observation that centhaquin improved the resuscitative effect of hypertonic saline (Gulati, Lavhale et al. 2012). It has been postulated that some of the cardiovascular effects of hypertonic saline are mediated through central nervous system (CNS) and centhaquin augments these effects (Gulati, Lavhale et al. 2012). In the present study, LR, which is not known to have a CNS-mediated resuscitative effect, was used and centhaquin continued to be an effective resuscitation agent, thereby indicating that its resuscitative effect may not be entirely mediated through the CNS.

Centhaquin was developed in 1970's as a centrally acting antihypertensive drug. However, because of a short duration of action, drug development was stopped following clinical phase I studies. The pharmacology of centhaquin is unique because it has positive inotropic action (Srimal, Mason et al. 1990) and sympatholytic action (Srimal, Gulati et al. 1990; Srimal, Mason et al. 1990; Gulati, Hussain et al. 1991). This positive inotropic action increases CO and MAP, while the sympatholytic action produces vasodilatation and a decrease in MAP.

It now has been found that centhaquin is an effective resuscitation agent having an effective dose for resuscitation at orders of magnitude lower (e.g., about 0.001 mg/kg) than the effective hypotensive dose (0.45 mg/kg). In particular, initial experiments in hemorrhaged rats with 0.45 mg/kg dose of centhaquin did not produce any increase in MAP of hemorrhaged rats, but produced a decrease in HR, an increase in CO, and a decrease in SVR. From these results, the sympatholytic action of centhaquin at higher doses was more prominent compared to that observed with lower doses. It is theorized, but not relied upon, that a low dose of centhaquin was not able to reach the central nervous system in sufficient amount for observable hypotension. Hence, it is the peripheral positive inotropic effect which dominated and produced an increase in MAP with the low doses used for resuscitation of hemorrhaged rats.

The administration of centhaquin dissolved in LR-100, which is one third the volume of LR-300, showed that centhaquin was a more effective resuscitation agent because it significantly improved CO and survival of rats compared to LR-300. In addition, aggressive fluid administration has been shown in animal models to increase bleeding because of increased arterial pressure, dilution of clotting factors, and decrease in blood viscosity (Kowalenko, Stem et al. 1992; Bickell, Wall et al. 1994). It has been advocated that if the "hypotensive resuscitation" (i.e., moderate increase in MAP) was performed using a small volume of resuscitation fluid, the possibility of dislodging the formed clot, acidosis, hypothermia, and coagulopathy was lower and could prevent worsening of hemorrhage (Watts, Trask et al. 1998; Engstrom, Schott et al. 2006; Kauvar, Lefering et al. 2006; Martini and Holcomb 2007; Morrison, Carrick et al. 2011). Because centhaquin was used in one-third the volume compared to LR-300 and an increase in MAP (about 20 to 25 mmHg) following resuscitation was moderate, additional blood loss should be minimal when resuscitation is performed with centhaquin compared to high volume LR -300 alone.

The exact mechanism of action of centhaquin is not known. However, initial studies showed that centhaquin (0.1, 1.0 and 10.0 µg/ml) produced an initial increase, followed by a decrease, of spontaneous release of norepinephrine and inhibited norepinephrine release evoked by potassium chloride and dimethyl phenyl piperazinium chloride (Bhatnagar, Pande et al. 1985). Therefore, a decrease in release of norepinephrine may lead to an increase in the density of α-adrenergic receptors, which may have improved vascular responsiveness. Spontaneous contraction of the right auricle of guinea pig was not affected by centhaquin up to a concentration of 2 µg/ml, but centhaquin significantly potentiated the positive inotropic effect of norepinephrine (Srimal, Mason et al. 1990). Centhaquin produced a marked increase in CO of hemorrhaged rats in the present study. In the terminal stages of hemorrhagic shock, progressive blood loss leads to a complex series of autonomic responses that include an initial increase in sympathetic drive which results in an overall increase in total peripheral resistance. However, after loss of about 20 to 30% blood volume, the sympathetic activity rapidly declines resulting in bradycardia, decrease in resistance and hypotension (Barcroft and Edholm 1945; Schadt and Ludbrook 1991). Centhaquin also has central sympatholytic activity (Murti, Bhandari et al. 1989; Srimal, Gulati et al. 1990) which can reduce the vasoconstriction caused by an initial increase in sympathetic drive following hemorrhagic shock. The hemodynamic pattern alone may not entirely explain the actions of centhaquin, and additional factors may contribute towards its resuscitative effects.

In conclusion, the tests described below show that centhaquin and its salts, in low doses, significantly improved CO, decreased blood lactate levels, and increased survival times in hemorrhaged rats and are useful drugs in emergencies.

In another study, the effect of centhaquin resuscitation on the amount of norepinephrine (NE) required to maintain mean arterial pressure (MAP) and the time by which MAP falls back to 35 mmHg in hemorrhaged rats was determined. Male, adult rats were anesthetized with urethane and a pressure catheter SPR-320 was placed in the femoral artery, and a pressure-volume catheter SPR-869 was placed in the left ventricle. Hemorrhage was induced by withdrawing blood and MAP was maintained at 35 mmHg for 30 minutes followed by resuscitation.

Two sets of experiments were performed. First, the amount of NE required to maintain MAP at 70 mmHg in normal saline (NS) or centhaquin (0.05 mg/kg) treated rats (volume equal to blood loss) was determined. Second, the time by which MAP fell back to 35 mmHg in rats treated with 3% hypertonic saline (HS) or centhaquin (0.05 mg/kg) (volume one-fifth of blood loss) was determined. Blood hematocrit decreased following hemorrhage and was similar in all the groups. Blood lactate was 4.10±1.02 mmol/L in NS compared to 1.65±0.23 mmol/L in centhaquin (P=0.041), 60 min following resuscitation. The amount of NE needed in each rat to maintain MAP at 70 mmHg was 175 µg in NS and 17.5 µg in centhaquin group during the first 60 min of resuscitation. In a second set of experiments, hemorrhaged rats were resuscitated with either HS or centhaquin (one-fifth the volume of blood loss). In centhaquin treated rats, blood lactate was 44% lower compared to hypertonic saline, and the time by which MAP fell back to 35 mmHg was 38±7 min in hypertonic saline compared to 148±15 min in centhaquin treated rats (P=0.0006). This was followed by blood transfusion (one-fifth the volume of blood loss), wherein hypertonic saline treated rats survived for 53±7 min compared to 78±8 min in centhaquin group (P=0.046). Centhaquin therefore is a highly effective resuscitation agent which decreases the requirement of NE in hemorrhaged rats, possibly due to improved vascular responsiveness. Centhaquin, with a low volume of resuscitation fluid, maintained MAP of hemorrhaged rats for a considerably long time and improved rat survival time.

Centhaquin also was found to increase the expression of endothelin A (ET_{A}) receptors in the blood vessels following intravenous administration in rats. Endothelin-1 (ET-1) increases the vascular reactivity of adrenergic receptors by acting on ET_{A} receptors (Tabuchi, Nakamaru et al. 1989; Gulati 1992; Gulati and Srimal 1993; Henrion and Laher 1993; Gulati and Rebello 1994; Sharma and Gulati 1995; Gondre and Christ 1998). Centhaquin therefore may have an adjunctive role in resuscitative effect by improving the vascular responsiveness in the resuscitation of hemorrhagic shock. The resuscitative effect of centhaquin was determined by adding centhaquin to Lactated Ringer's or hypertonic saline in rats with hemorrhagic shock. Rats were anaesthetized with urethane. The femoral vein was cannulated for drug administration and femoral artery was cannulated for measuring mean arterial pressure (MAP). A calibrated pressure-volume catheter (SPR-869) was placed into the left ventricle through the right carotid artery. Induction of hemorrhagic shock was initiated by withdrawing blood to maintain the MAP between 35 and 40 mmHg for 30 minutes. The animals were divided into four groups: Group I, Lactated Ringer's solution, volume equal to shed blood volume (SBV): Group II, Lactated Ringer's solution, volume equal to SBV plus centhaquin 0.05 mg/kg: Group III, 3% hypertonic saline (HS), volume equal to SBV: Group IV, 3% hypertonic saline, volume equal to SBV plus centhaquin 0.05 mg/kg. Data at 30 min after HS and 60 min following resuscitation are summarized in the table below:

| | Time | Group I (LR only) | Group II (LR + Centhaquin) | Group III (3% hypertonic saline) | Group IV (3% hypertonic saline + Centhaquin) |
|---|---|---|---|---|---|
| Blood Lactate (mmol/L) | Hemorrhagic shock | 7.2±0.7 | 7.1±0.4 | 7.4±0.4 | 7.3±0.4 |
| | Resuscitation | 10.2±0.6 | 4.1±0.3 | 3.4±0.5 | 2.0±0.3 |
| Mean Arterial Pressure (mmHg) | Hemorrhagic shock | 34.0±3.2 | 34.1±3.7 | 35.3±3.3 | 36.1±3.9 |
| | Resuscitation | 24.2±3.5 | 53.9±4.2 | 35.1±4.5 | 60.1±4.1 |
| Cardiac Output (mL/min) | Hemorrhagic shock | 29.0±5.6 | 27.4±5.1 | 39.7±4.4 | 36.4±4.5 |
| | Resuscitation | 20.7±6.0 | 76.8±3.6 | 96.2±4.9 | 116.8±4.5 |
| Systemic Vascular Resistance (mmHg/µl/min) | Hemorrhagic shock | 0.001±0.00 01 | 0.001±0.00006 | 0.0008±0.0001 | 0.0009±0.00002 |
| | Resuscitation | 0.001±0.00 01 | 0.0007±0.00007 | 0.0003±0.0001 | 0.0005±0.00002 |
| Survival Time (min) | | 78±9 | 387±38 | 144±22 | 326±55 |

The data in the above table show that survival improved significantly (p=<0.001) with the addition of centhaquin to the resuscitation fluid, either LR or hypertonic saline. Similarly blood lactate was significantly lower when centhaquin was added to the resuscitation fluid. The results show that the addition of centhaquin to a resuscitation fluid improves cardiovascular functions and survival.

As demonstrated above, centhaquin improves cardiac output, tissue oxygenation, and organ perfusion, and is useful in the treatment of diseases and conditions in which such improvements are needed, including hemorrhagic shock. These benefits provided by centhaquin are attributed to its activity as an α₂ adrenergic agent, and accordingly, other known α₂ adrenergic agents are likewise expected to provide the beneficial results.

Therefore, although the above data is presented for a specific α₂ adrenergic agent, the methods of the present invention also can be accomplished by the administration of an α₂ adrenergic agent selected from the group consisting of centhaquin, clonidine, guanfacine, guanabenz, guanoxabenz, guanethidine, xylazine, tizanidine, methyldopa, fadolmidine, amidephrine, amitraz, anisodamine, apraclonidine, brimonidine, cirazoline, detomidine, dexmedetomidine, epinephrine, ergotamine, etilefrine, indanidine, lofexidine, medetomidine, mephentermine, metaraminol, methoxamine, mivazerol, naphazoline, norepinephrine, norfenefrine, octopamine, oxymetazoline, phenylpropanolamine, rilmenidine, romifidine, synephrine, talipexole, salts thereof, and mixtures thereof.

In addition to the α₂ adrenergic agents disclosed herein, salts of the α₂ adrenergic agents also can be used in the present methods. Examples of suitable salts include, but are not limited to, acid addition salts formed with inorganic acids such as nitric, boric, hydrochloric, hydrobromic, sulfuric, and phosphoric, and organic acids such as oxalic, maleic, succinic, tartaric, and citric. Nonlimiting examples of salts of α₂ adrenergic agents include, but are not limited to, the hydrochloride, hydrobromide, hydroiodide, sulfate, bisulfate, 2-hydroxyethansulfonate, phosphate, hydrogen phosphate, acetate, adipate, alginate, aspartate, benzoate, bisulfate, butyrate, camphorate, camphorsulfonate, digluconate, glycerolphosphate, hemisulfate, heptanoate, hexanoate, formate, succinate, fumarate, maleate, ascorbate, isethionate, salicylate, methanesulfonate, mesitylenesulfonate, naphthylenesulfonate, nicotinate, 2-naphthalenesulfonate, oxalate, pamoate, pectinate, persulfate, 3-phenylproprionate, picrate, pivalate, propionate, trichloroacetate, trifluoroacetate, phosphate, glutamate, bicarbonate, undecanoate, lactate, citrate, tartate, gluconate, methanesulfonate, ethanedisulfonate, benzene sulfonate, and p-toluenesulfonate salts.

Preferred salts are salts of organic acids, such as citrate, tartrate, malate, succinate, oxalate, fumarate, maleate, ascorbate, lactate, gluconate, diglyconate, and aspartate, for example. A more preferred salt is a citrate salt, a lactate salt, or a tartrate salt.

As demonstrated above, centhaquin is administered at a low dosage to achieve the benefits of the present methods. Therefore, centhaquin, as the free base, is administered in an amount of 0.001 to less than 0.05 mg per kg of weight of the individual being treated (mg/kg), preferably about 0.003 to about 0.04 mg/kg, and more preferably about 0.005 to about 0.03 mg/kg.

More particularly, centhaquin, as the free base, is administered at mg/kg doses of 0.001, 0.002, 0.003, 0.004, 0.005, 0.006, 0.007, 0.008, 0.009, 0.010, 0.011, 0.012, 0.013, 0.014, 0.015, 0.016, 0.017, 0.018, 0.019, 0.020, 0.021, 0.022, 0.023, 0.024, 0.025, 0.026, 0.027, 0.028, 0.029, 0.030, 0.031, 0.032, 0.033, 0.034, 0.035, 0.036, 0.037, 0.038, 0.039, 0.040, 0.041, 0.042, 0.043, 0.044, 0.045, 0.046, 0.047, 0.048, or 0.049, and all ranges and subranges therein.

As demonstrated below, centhaquin also can be administered in the form of salt, e.g., centhaquin citrate, to achieve the benefits of the present methods. Centhaquin citrate is administered in an amount of about 0.0001 to about 1.5 mg/kg, preferably about 0.0002 to about 0.8 mg/kg, and more preferably about 0.0004 to about 0.5 mg/kg. More particularly, centhaquin citrate can be administered at mg/kg doses (as centhaquin citrate) of 0.001, 0.002, 0.003, 0.004, 0.005, 0.006, 0.007, 0.008, 0.009, 0.010, 0.011, 0.012, 0.013, 0.014, 0.015, 0.016, 0.017, 0.018, 0.019, 0.020, 0.021, 0.022, 0.023, 0.024, 0.025, 0.026, 0.027, 0.028, 0.029, 0.030, 0.031, 0.032, 0.033, 0.034, 0.035, 0.036, 0.037, 0.038, 0.039, 0.040, 0.041, 0.042, 0.043, 0.044, 0.045, 0.046, 0.047, 0.048, 0.049, 0.05, 0.051, 0.052, 0.053, 0.054, 0.055, 0.06, 0.065, 0.07, 0.075, 0.08, 0.085, 0.09, 0.095, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.1, 1.2, 1.3, 1.4, or 1.5, and all ranges and subranges therein.

Based on the molecular weight of centhaquin (free base) (MW-332) and centhaquin citrate (MW-523), for identical doses of centhaquin (as free base) and centhaquin citrate, centhaquin citrate provides only 63.5% of centhaquin free base compared to the dose of centhaquin free base, e.g., a 0.05 mg dose of centhaquin citrate contains a 0.0318 mg of centhaquin (as free base). Similarly, a dose of centhaquin citrate dihydrate (MW-559) provides 59.4% centhaquin (free base) of the same dose as centhaquin (as free base), i.e., a 0.0005 mg dose of centhaquin citrate dihydrate contains 0.030 mg of centhaquin (as free base). Surprisingly, and as demonstrated below, at the same mg/kg dose centhaquin citrate and centhaquin citrate dihydrate provides greater cardiovascular effects than centhaquin free base.

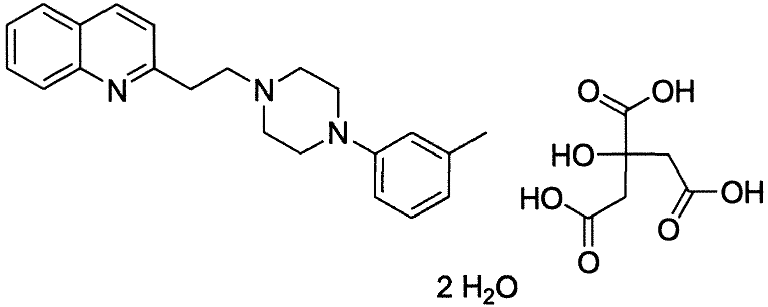

### Structure of centhaquin citrate dihydrate

The α₂ adrenergic agent typically is coadministered with a resuscitation fluid. The resuscitation fluid can be a colloid solution, a crystalloid solution, blood, a blood component or a blood substitute. Nonlimiting examples of colloid solutions and crystalloid solutions are Ringer's Lactate, saline, hypertonic saline, an albumin solution, a dextran solution, a hydroxyethyl starch solution, a gelatin solution, and a starch solution. Examples of a blood component are plasma, red blood cells, white blood cells, platelets, clotting factors, proteins, and hormones. The blood substitute can be a hemoglobin-based blood substitute or a perflourocarbon-based substitute.

The resuscitation fluid can administered in a volume amount of up to three times the volume amount of fluid, e.g., blood, plasma, water, lost by an individual. In preferred embodiments, the resuscitation fluid is administered in a volume amount less than and up to the volume amount of fluid lost by the individual, e.g., a volume amount of 5%, preferably 10% or 20%, and up to 100% of the volume amount of lost fluid.

In accordance with preferred embodiments of the invention, the α₂ adrenergic agent administered to an individual is centhaquin or centhaquin citrate. In more preferred embodiments, the centhaquin is a purified centhaquin having a melting point of 94 ± 2°C or a purified centhaquin citrate having a melting point of 94 ± 2°C. A hydrate of centhaquin citrate also can be administered, e.g., a 0.5 hydrate, 0.7 hydrate, monohydrate, or dihydrate.

### Improved method to prepare and purify centhaquin and centhaquin citrate

### Synthesis of Centhaquin

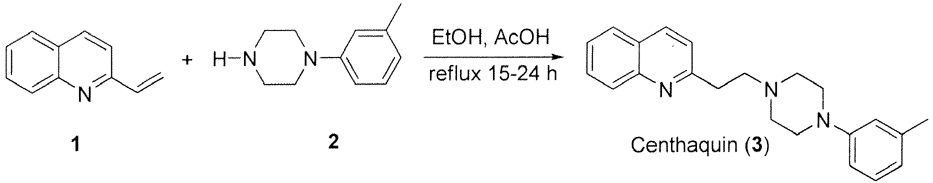

The synthesis of centhaquin was reported by Murthi and coworkers (Murthi et al. U.S. Patent No. 3,983,121; Murti, Bhandari et al. 1989). In one procedure, reactants 1 and 2 were stirred at reflux for 15 hours. The resulting product was purified by evaporating the solvents to obtain an oil, which was heated *in vacuo* (100°C, 1 mm Hg). The remaining residue was recrystallized from ether-petroleum ether to obtain the final centhaquin product 3. The melting point reported for centhaquin was 76-77°C. In a subsequent publication (Murti, Bhandari et al. 1989), the reaction mixture was concentrated following 24 hours of reflux, diluted with water, and basified with aqueous NaOH. The basic mixture was extracted with ethyl acetate, and the ethyl acetate extracts were dried over anhydrous sodium sulfate and evaporated *in vacuo* to give centhaquin which was crystallized from hexane. The melting point of centhaquin (free base) obtained in this procedure was 82°C. The product obtained using either purification method is light tan in color, which is indicative of small amounts of impurities that were not completely removed using previously reported purification methods.

An improved purification method was found. According to the improved method, reactants 1 and 2 were stirred at reflux for 24 hours. The solvents were evaporated *in vacuo* and the resulting mixture was diluted with water and basified (10% NaOH). The basic mixture was extracted with ethyl acetate and the combined ethyl acetate extracts are dried over anhydrous sodium sulfate and evaporated *in vacuo* to obtain a residue, which was further purified with column chromatography (SiO₂, ethyl acetate). The resulting product can be decolorized using activated charcoal or directly crystallized from hot hexane to yield pure centhaquin. The resulting product is an off-white crystalline solid having a melting point of 94-95°C (free base). The product was characterized using proton NMR, mass spectral, and elemental analysis and indicated high purity and superior quality.

Synthesis and characterization of centhaquin (free base): A mixture of 2-vinylquinoline (1) (5.0 g, 32.2 mmol, 98.5%) and 1-(3-methylphenyl)piperazine (2) (5.68 g, 32.2 mmol, 99.0%) in absolute ethyl alcohol (150 ml) and glacial acetic acid (3.5 ml) was stirred at reflux for 24 hours in a round bottom flask. The reaction mixture was concentrated *in vacuo,* diluted with water (150 ml) and treated with 10% aqueous NaOH (150 ml). The residue was extracted with ethyl acetate (4 x 125 ml), dried with anhydrous Na₂SO₄, and concentrated under reduced pressure to yield a crude product which was purified by column chromatography using silica gel (100-200 mesh) with ethyl acetate as an eluent. The resulting compound was recrystallized from hot hexane and filtered, to yield centhaquin as an off-white crystalline solid (7.75 g, 23.4 mmol, 73% yield); mp. 94-95°C; R*_{f}*0.30 (100% ethyl acetate); ¹H NMR (300 MHz, CDCl₃): δ 8.07 (t, *J* = 7.5 Hz, 2 H), 7.78 (d, *J* = 7.8 Hz, 1H), 7.70 (t, *J* = 7.8 Hz, 1H), 7.50 (t, *J* = 7.5 Hz, 1H), 7.36 (d, *J* = 8.4 Hz, 1H), 7.16 (t, *J* = 7.5 Hz, 1H), 6.77 - 6.74 (m, 2 H), 6.69 (d, *J* = 7.2 Hz, 1H), 3.26- 3.21 (m, 6 H), 2.97 - 2.92 (m, 2 H), 2.76 - 2.73 (m, 4 H), 2.32 (s, 3 H); HRMS (ESI) m/z 332.2121 [M+1]⁺ (calcd for C₂₂H₂₆N₃ 332.2122); Anal. (C₂₂H₂₅N₃) C, H, N.

Preparation of centhaquin citrate: Centhaquin (free base) (5.62 g, 16.98 mmol) was treated with citric acid (3.26 g, 16.98 mmol) in a suitable solvent and converted to the citrate salt obtained as an off-white solid (7.96 g, 15.2 mmol, 90%); m.p. 94-96°C; Anal. (C₂₈H₃₃N₃O₇.2H₂O) C, H, N.

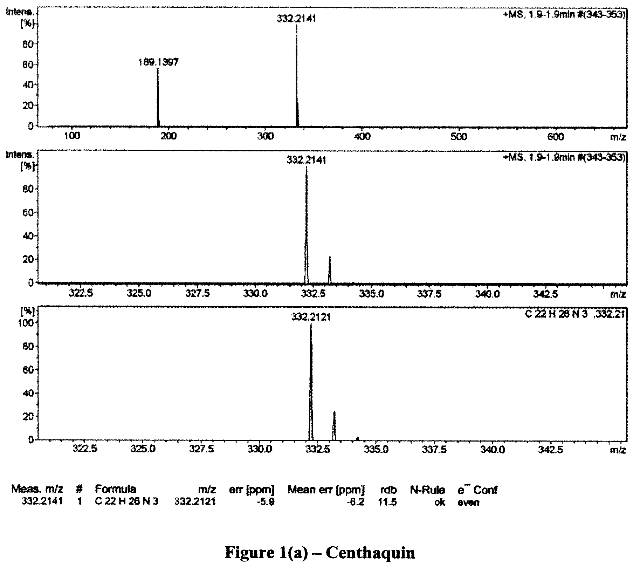

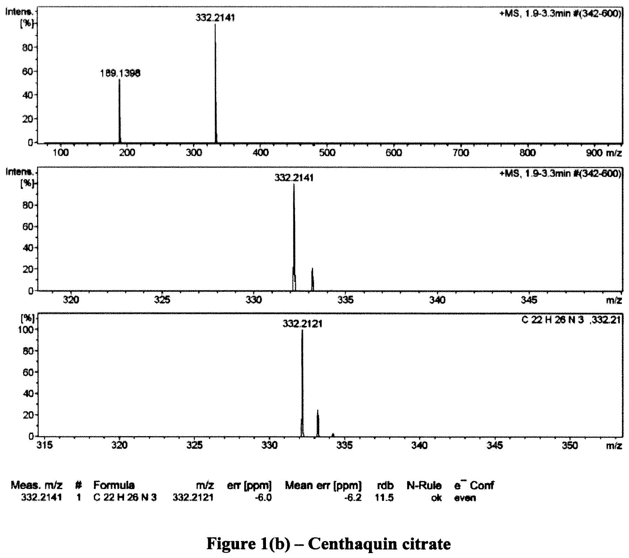

Figs. 1(a) and 1(b) are high resolution mass spectral analyses of centhaquin free base (Fig 1(a)) and centhaquin citrate (Fig. 1(b)). Compound samples were analyzed following ionization using electrospray ionization (ESI).

For centhaquin free base in Fig 1(a), a base peak [M+1]⁺ was observed at m/z 332.2141 (theory: 332.2121) consistent with the elemental composition of protonated centhaquin (C₂₂H₂₆N₃)·

For centhaquin citrate in Fig 1(b), the mass spectrum was identical to the mass spectrum obtained for the free base. An [M+1]⁺ base peak was observed at m/z 332.2141 (theory: 332.2121), which corresponds to the elemental composition of protonated centhaquin (C₂₂H₂₆N₃). This result is typical of salts of organic bases to yield the [M+1]⁺ of the free base as observed here with centhaquin citrate.

Mass spectrometry is one of the most sensitive analytical methods, and examination of the mass spectra of Fig. 1 indicate that the samples are devoid of any extraneous peaks and are of homogeneous purity (>99.5).

The attached figures illustrate the efficacy of centhaquin and centhaquin citrate in treating diseases and conditions where an improvement in cardiac output, tissue oxygenation, and oxygen perfusion provides a benefit, i.e., in reversing the end points of shock. In the following figures, the amount of drug dosed is centhaquin (as free base) and centhaquin citrate (as the citrate). As discussed above, the amount of centhaquin (as free base) in a dose of centhaquin citrate is only 63.5% of the amount of centhaquin in the same dose of centhaquin as free base, i.e., a 5 mg dose of centhaquin as free base is a 5 mg dose of centhaquin free base, whereas a 5 mg dose of centhaquin citrate is equivalent to a 3.1 mg dose of centhaquin as a free base. Similarly, a 5 mg dose of centhaquin citrate dihydrate is equivalent to a 3.0 mg dose of centhaquin (free base), i.e., 59.4%.

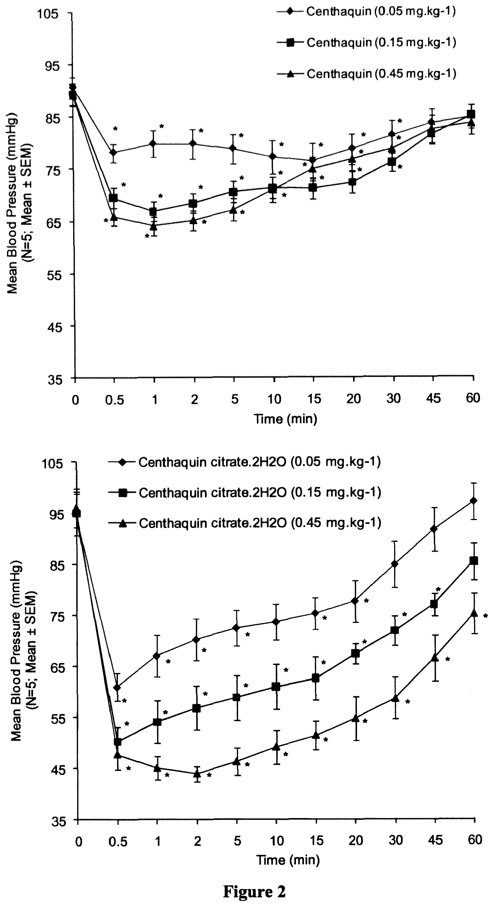

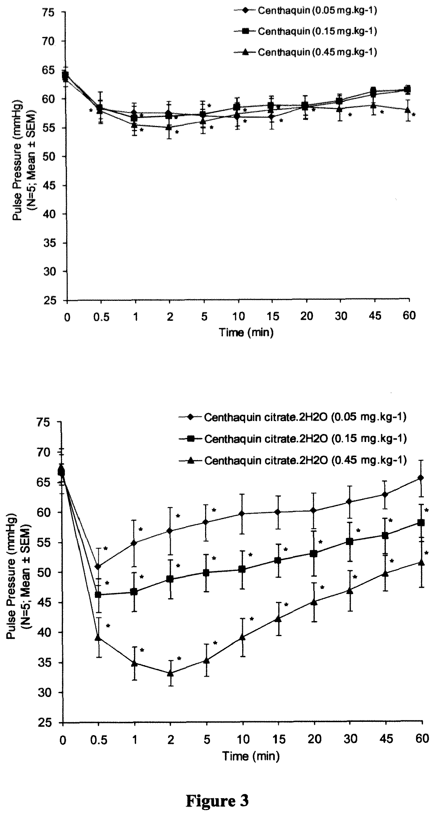

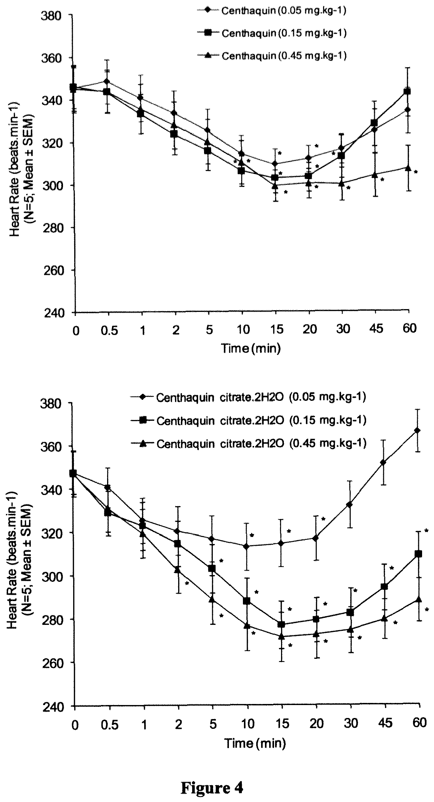

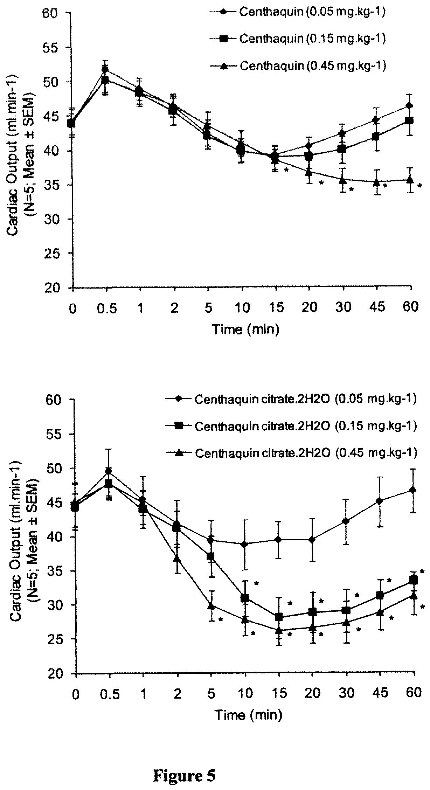

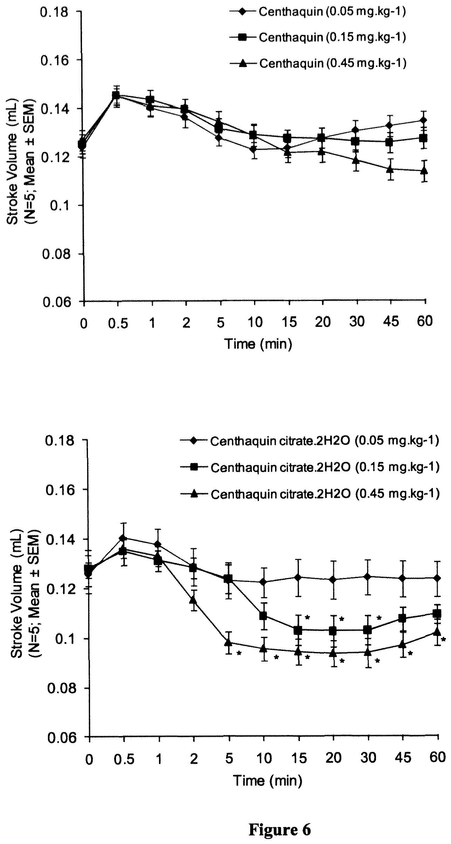

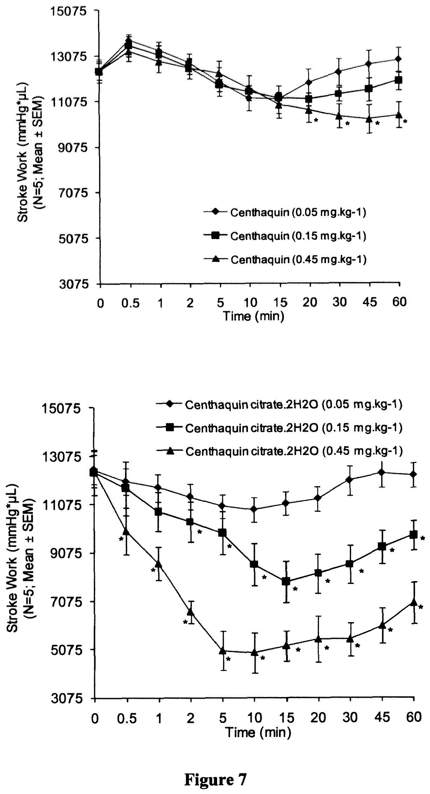

As demonstrated in the attached figures, a mg/kg dose of centhaquin citrate greatly outperforms the same mg/kg dose of centhaquin, although the dose of centhaquin citrate provides only 63% of centhaquin free base as the centhaquin free base dose. This unexpected and unpredictable result is shown in Figures 2 through 7.

Figures 2 through 7 contain plots of various cardiovascular effects demonstrated by centhaquin and centhaquin citrate over time. In these plots, centhaquin is tested as the free base in amounts of 0.05, 0.15, and 0.45 mg/kg. Similarly, centhaquin citrate dihydrate is tested in amounts of 0.05, 0.15, and 0.45 mg/kg. These amounts of centhaquin citrate are equivalent to 0.030, 0.089, and 0.267 mg/kg of centhaquin as the free base.

Figures 2 through 7 show that centhaquin and centhaquin citrate provide positive cardiovascular effects of mean blood pressure, pulse pressure, heart rate, cardiac output, stroke volume, and stroke work in urethane anesthetized rats over a 60 minute time period. Surprisingly, centhaquin citrate, at a centhaquin free base amount substantially lower than an amount of administered centhaquin free base, substantially outperformed centhaquin free base with respect to cardiovascular effects.

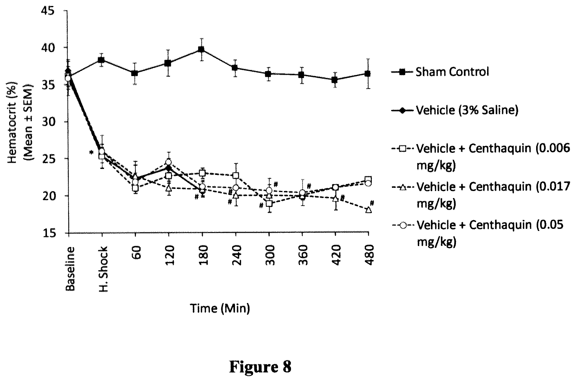

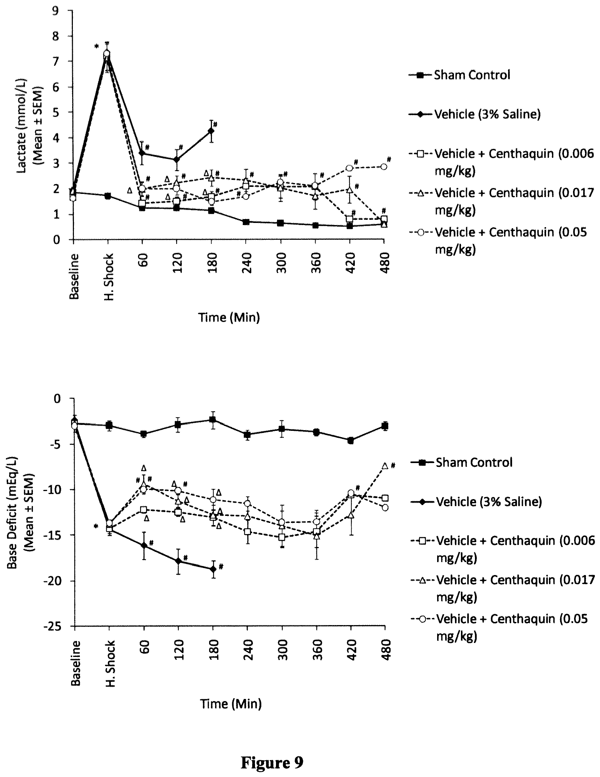

Figure 8 shows the effect of centhaquin (free base) on blood hematocrit in hemorrhaged rats. A sham group of rats was cannulated, but not hemorrhaged. A vehicle group received hypertonic saline. Treatment groups received centhaquin (free base) (0.006, 0.017, or 0.05 mg/kg) in hypertonic saline. The values are expressed as mean ± S.E.M. with n=6 rats in each group. The data in Figure 8, and in following Figures 9-13, show that very low doses of centhaquin (free base) have a positive effect on cardiovascular effects.

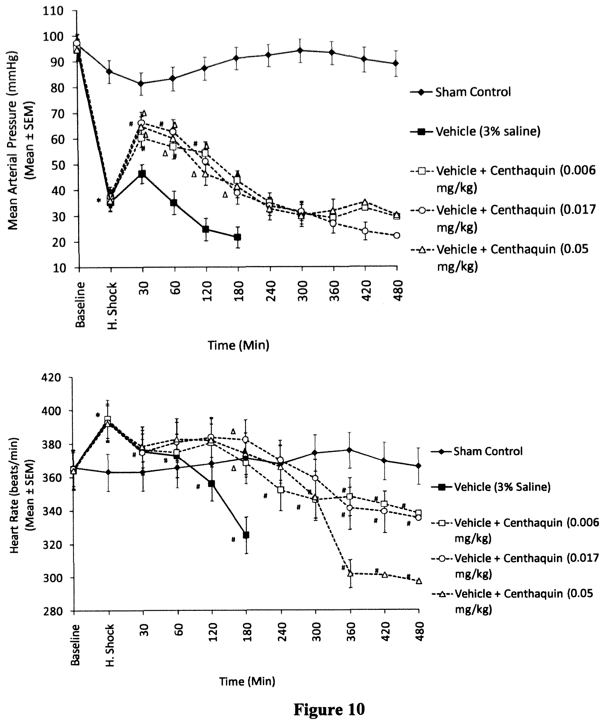

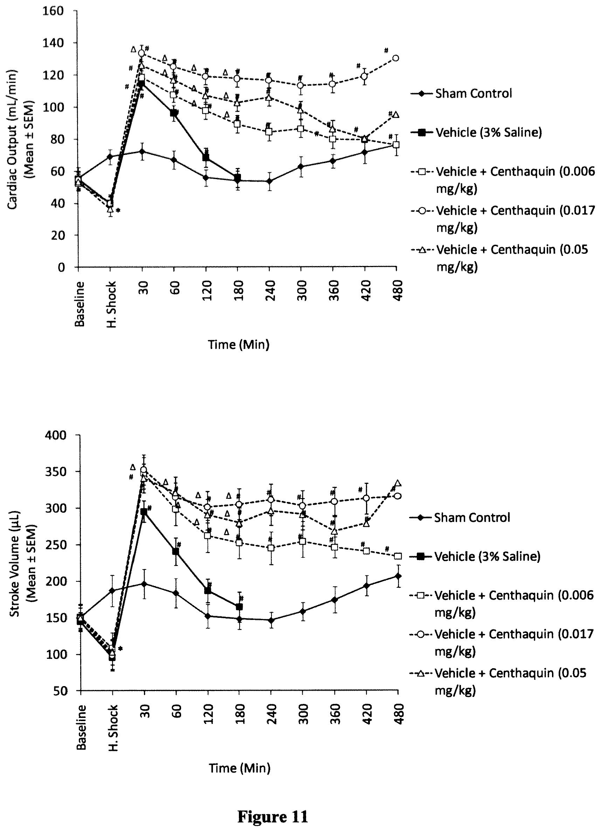

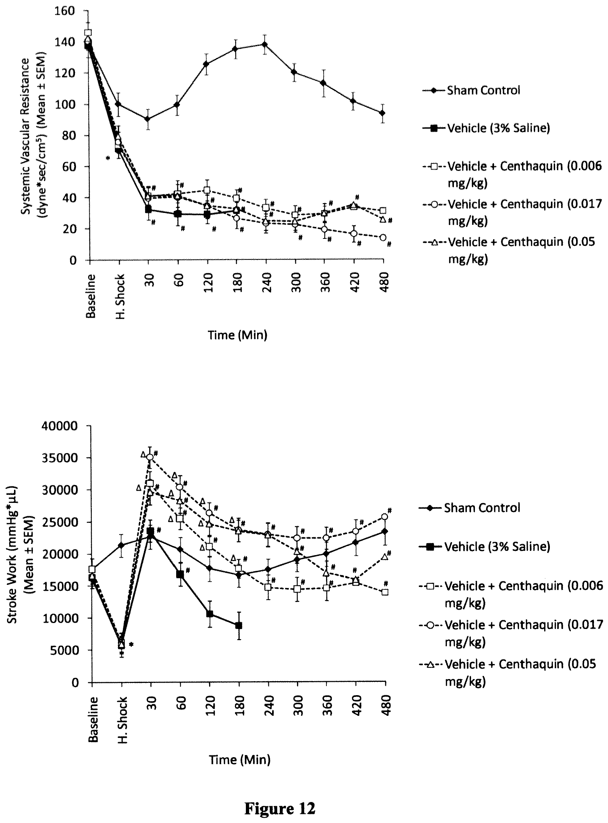

Figures 9-12 show the effect of centhaquin on blood lactate and base deficit in hemorrhaged rats (Figure 9); on mean arterial pressure and heart rate (Figure 10); on cardiac output and stroke volume (Figure 11); and on systemic vascular resistance and stroke work (Figure 12). Like the tests of Figure 8, a sham group of rats was cannulated but not hemorrhaged, a vehicle group received hypertonic saline, and treatment groups received free base centhaquin (0.006, 0.017, or 0.05 mg/kg) in hypertonic saline. The values are expressed as mean ± S.E.M. with n=6 rats in each group. *p<0.05 compared to baseline, #p<0.05 compared to hemorrhage, Δp<0.05 compared to vehicle treated group.

Such positive cardiovascular effects make centhaquin an excellent resuscitation agent for reversing the end points of shock. Importantly, by the administration of centhaquin, centhaquin citrate or hydrate thereof, or other α₂ adrenergic agent or salt thereof, the volume amount of a resuscitation fluid can be reduced, e.g., use of LR-100 as opposed to LR-300, which eliminates the adverse effects attributed to using a high volume of resuscitation fluid

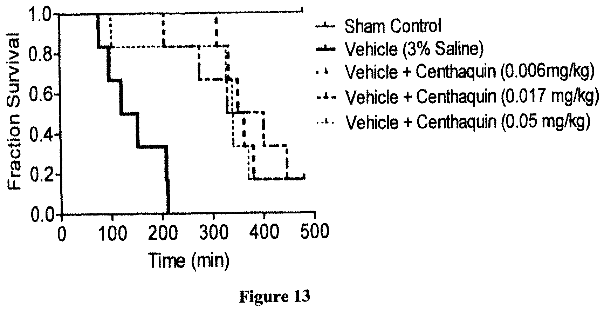

Figure 13 is a plot of fraction survival vs. time for groups of hemorrhaged rats. The sham group of rats was cannulated, but not hemorrhaged. The vehicle group received hypertonic saline, while treatment groups received centhaquin (free base) (0.006, 0.017, or 0.05 mg/kg) in hypertonic saline.

### METHODS OF TREATMENT

### Hypovolemic shock

Hypovolemic shock results in a reduction of blood or fluid volume, which can be attributed to blood loss (internal and external), dehydration, diarrhea, burns, and vomiting, for example. Hypovolemic shock is caused by the loss of both circulating blood volume and oxygen-carrying capacity. Shock due to severe hemorrhage accounts for a large portion of posttraumatic deaths, and is a major causative factor in almost half of the deaths on the battlefield (Wu, Dai et al. 2009). Most of the deaths due to hemorrhage occur in the first six hours after trauma (Shackford, Mackersie et al. 1993) and many of these deaths can be prevented (Bellamy 1984; Acosta, Yang et al. 1998).

Hemorrhagic shock is a serious state that results from excessive blood loss and inadequate oxygen perfusion, which fails to sustain the physiologic needs of organ tissues and can lead to hemodynamic instability. In shock, blood loss exceeds the body's ability to compensate and provide adequate tissue perfusion and oxygenation. In the initial stages of hypovolemia, physiologic compensatory mechanisms redistribute cardiac output and blood volume. The heart plays a critical role in compensation for losses in early shock, and responses are associated with reflex tachycardia and increased stroke volume. There is intense vasoconstriction in the skin, skeletal muscle, and splanchnic circulation. Coronary, renal, and cerebral circulations do not experience an increase in vascular resistance in early shock. Therefore, cardiac output is shunted to vital organs in the initial stages of shock.

At a later stage, shock is accompanied by circulatory failure which is mainly responsible for mortality and morbidity. It is imperative therefore that hemodynamically unstable patients are supported and resuscitated. Shock triggers multiple compensatory mechanisms to preserve oxygenation and tissue blood flow, such as activation of the sympathetic nervous system, alteration in cardiac functions, hormonal changes, renal volume, and electrolyte imbalance (Heslop, Keay et al. 2002; Liu, Ward et al. 2003; Hardy, de Moerloose et al. 2006; Pfeifer, Kobbe et al. 2011).

Hemorrhaged patients are resuscitated with intravenous crystalloid fluids to restore oxygen delivery. However, patients may develop irreversible loss of capillary bed perfusion, coagulopathy, hypothermia, acidosis, immune suppression, systemic inflammation, oxidative stress, multiple organ failure, and death (Bickell, Bruttig et al. 1991; Rhee, Burris et al. 1998; Heslop, Keay et al. 2002; Liu, Ward et al. 2003; Dubick, Bruttig et al. 2006; Hardy, de Moerloose et al. 2006; Alam and Rhee 2007; Pfeifer, Kobbe et al. 2011).

Resuscitation with a crystalloid solution, a colloid solution, or packed red blood cells dilutes plasma coagulation factors and exacerbates hemorrhage (Hardy, de Moerloose et al. 2006; Malone, Hess et al. 2006). The two most commonly used isotonic crystalloid solutions in emergency departments and prehospital settings are Lactated Ringer's (LR) and normal saline (NS). These fluids possess pH ranges as low as 4.5 for NS and 6.0 for LR. Using large amounts NS in trauma patients with shock contributes to metabolic acidosis which can worsen coagulopathy (Ho, Karmakar et al. 2001) leading to further hemorrhage, necessitating additional fluid resuscitation which contributes to more profound coagulopathy due to hemodilution and hypothermia. Hemorrhagic shock and resuscitation also is associated with development of inflammation, oxidative stress, and apoptosis leading to multiple organ failure and death (Ganster, Burban et al. 2010; Hsia and Ma 2011; Zacharias, Sailhamer et al. 2011).

The current recommended fluid therapy of using large volumes of LR is effective in restoring hemodynamic parameters, but presents logistic and physiologic limitations (Vincenzi, Cepeda et al. 2009). Resuscitation with large volume of crystalloids has been associated with secondary abdominal compartment syndrome, pulmonary edema, cardiac dysfunction, and paralytic ileus (Balogh, McKinley et al. 2003). It has even been proposed that LR may be detrimental in patients with uncontrolled hemorrhage (Bickell, Bruttig et al. 1991; Bickell, Bruttig et al. 1992). Reportedly, LR exacerbates neutrophil superoxide burst activity and increases neutrophil adherence (Rhee, Burris et al. 1998). Aggressive resuscitation with crystalloids led to increased cytokine activation including IL-1, IL-6, and TNF (Hierholzer, Harbrecht et al. 1998). LR also activates the immune system and may contribute to secondary cellular injury (Rhee, Koustova et al. 2003; Alam, Stanton et al. 2004; Ayuste, Chen et al. 2006; Watters, Tieu et al. 2006).

Large volume resuscitation has been used by emergency medical personnel and surgeons to reverse hemorrhagic shock and to restore end-organ perfusion and tissue oxygenation. However, there has been a vigorous debate about the optimal methods of resuscitation (Santry and Alam 2010). It is becoming increasingly clear that the desired method of resuscitation will involve using a small volume rather than large volume of resuscitation fluid and maintaining a blood pressure that is slightly below normal, which appears to produce better clinical outcomes (Dries 1996; Gulati, Sen et al. 1997; Drabek, Kochanek et al. 2011; Morrison, Carrick et al. 2011). The present invention allows the use of low volume of resuscitation fluid by the coadministration of a low dose of an α₂ adrenergic agent.

Initial experiments in hemorrhaged rats were performed with a 0.45 mg/kg dose of centhaquin. At this dose, centhaquin (as free base) did not produce an increase in MAP of hemorrhaged rats and MAP (mmHg) was found to be 34 ± 3 at hemorrhage, and 38 ± 4, 39 ± 4 and 35 ± 4 at 30, 60 and 120 min, respectively, following resuscitation. Centhaquin (0.45 mg/kg) produced a decrease in HR (beats/min) from 368 ± 12 at hemorrhage, to 306 ± 6, 309 ± 6 and 274 ± 11 at 30, 60 and 120 min, respectively, following resuscitation; cardiac output (mL) increased from 32 ± 6 at hemorrhage, to 77 ± 3, 74 ± 4 and 62 ± 5 at 30, 60 and 120 min, respectively, following resuscitation; while systemic vascular resistance (dynes*sec/cm5) was 84 ± 4 at hemorrhage, and fell to 39 ± 9,42 ± 9 and 45 ± 6 at 30, 60 and 120 min, respectively, following resuscitation. From these results, at higher doses, the sympatholytic action of centhaquin was more prominent compared to that observed with lower doses.

In accordance with the present invention centhaquin citrate (or a solvate thereof) can be administered as a resuscitation agent at doses orders of magnitude lower (e.g., about 0.001 mg/kg) than the hypotensive dose (0.45 mg/kg). Low doses of centhaquin and centhaquin citrate produced an increase in blood pressure when infused intravenously, while higher doses produced a fall in blood pressure. It was found that low doses of centhaquin were more effective in resuscitation because at a low dose, centhaquin is not able to reach the central nervous system in the amount needed to produce observable hypotension. Hence, it is the peripheral positive inotropic effect of centhaquin that predominates and produces an increase in mean arterial pressure at the low doses of centhaquin used for resuscitation of hemorrhaged rats.

Centhaquin (0.05 to 0.2 mg/kg, iv) produced a dose-dependent decrease in mean arterial pressure (MAP) and heart rate (HR) in urethane anesthetized rats and conscious freely moving cats and rats (Srimal, Gulati et al. 1990). Evidence for its central site of action has been reported earlier (Gulati, Hussain et al. 1991). Centhaquin consistently reduced blood lactate levels from 2.42 ± 0.17 to 1.25 ± 0.55 mg/dL in normal rats, and it has been established that the odds of hyperlactatemia by logistic regression analysis are 4.6 (95% confidence interval 1.4-15; p < 0.05) times higher for a patient with abnormal peripheral perfusion (Lima, Jansen et al. 2009). Elevated lactate levels are associated with a worse prognosis for trauma patients. Inadequate oxygen delivery, disproportionate oxygen demand, and diminished oxygen use may lead to elevated lactate levels. Blood lactate monitoring is recommended in critical care settings and clearly has a place in the risk-stratification of critically ill patients (Jansen, van Bommel et al. 2009). Because centhaquin reduces blood lactate levels, it provides a resuscitative effect in hemorrhagic shock.

Agmatine, an endogenous substance that acts on sites similar to the α₂ adrenergic agent clonidine, also was found to be an effective resuscitation agent in hemorrhaged rats. The resuscitative effect of agmatine was completely blocked by yohimbine indicating involvement of α₂-adrenergic receptors (Gill, Pelit et al. 2011). By decreasing central nervous system sympathetic output, a decrease in systemic vascular resistance and an increase in cardiac output and end organ perfusion occurs. Centhaquin also has central sympatholytic activity (Murti, Bhandari et al. 1989; Srimal, Gulati et al. 1990) and it may reduce vasoconstriction caused by an increase in sympathetic drive following hemorrhagic shock. Thus, centhaquin increases cardiac output and improves regional blood circulation through its sympatholytic action, thereby protecting the organs from failure in hemorrhagic shock. Centhaquin-induced increase in cardiac output was much more pronounced compared to a decrease in systemic vascular resistance. Therefore, the central sympatholytic action of centhaquin is less prominent at the low doses used for resuscitation, and it is primarily an increase in cardiac output that contributes to improved blood circulation and survival of hemorrhaged rats.

### Resuscitative effect of centhaquin in hemorrhagic shock

A rodent model of fixed-pressure hemorrhage without tissue trauma was used in a preliminary study. This hemorrhage model was made more severe by maintaining the hypotension for 30 min from the onset of hemorrhage to reach a base deficit of greater than - 12 mEq/L (Gulati, Sen et al. 1997). Hematocrit decreased as a result of blood loss and it was similar in all the groups undergoing resuscitation. Following groups were studied: Lactated Ringer's solution (LR-100) (100% shed blood volume (SBV)), or centhaquin (free base) (0.017,0.05 and 0.15 mg/kg) dissolved in LR (100% SBV), or LR-300 (300% SBV). It was found that survival time following resuscitation with LR-100 was 78±10 min. Centhaquin in doses of 0.017 and 0.05 mg/kg significantly improved survival time to 291±57 and 387±39 min, respectively. Blood lactate levels (mmol/L) increased from 7.22±0.67 at hemorrhage to 10.20±0.61 at 60 min following resuscitation with LR-100. On the other hand, blood lactate levels significantly decreased to 3.55±0.07 and 4.08±0.28 at 60 min following resuscitation with 0.017 and 0.05 mg/kg dose of centhaquin, respectively. Centhaquin in doses of 0.017 and 0.05 mg/kg produced a 55% and 59% increase in MAP, respectively compared to a decrease of 29% by LR-100. A decrease in systemic vascular resistance by 57% and 41% was observed with 0.017 and 0.05 mg/kg doses of centhaquin, compared to a 6% decrease by LR-100. Cardiac output decreased by 28% with LR-100, whereas 0.017 and 0.05 mg/kg doses of centhaquin increased cardiac output by 260% and 180%, respectively. Compared to LR-300, centhaquin (0.05 mg/kg) improved survival time, increased cardiac output, and was more effective in resuscitation of hemorrhaged rats. Centhaquin therefore was found to be a highly effective resuscitation agent for the treatment of hemorrhagic shock in rat.

It previously was found that hypertonic saline had a resuscitative effect in hemorrhaged rats. It now has been found that centhaquin markedly enhances the resuscitative effect of hypertonic saline. Centhaquin significantly decreased blood lactate, and increased MAP and cardiac output compared to hypertonic saline alone. Centhaquin increased the survival time of hemorrhaged rats compared to hypertonic saline treatment. Median survival time for 50% survival rate of rats treated with hypertonic saline was 137 ± 12 minutes, while for rats treated with centhaquin it was 375 ± 25 minutes. Fraction survival at 250 min was 0 when resuscitated with hypertonic saline, while treatment with centhaquin improved the fraction survival to 0.8. Even at 480 min after resuscitation with centhaquin, the fraction survival was 0.2. Rats treated with 0.017 mg/kg dose of centhaquin had the maximal survival time compared to other groups. The findings of the present study are novel, unexpected, and are important due to recent reports of large clinical studies showing hypertonic saline has no benefit over normal saline in patients with hypovolemic shock or severe traumatic brain injury (Bulger, May et al. 2010; Bulger, May et al. 2011). The present results demonstrate that centhaquin can augment the resuscitative effect of hypertonic saline in the treatment of such conditions.

### Effect of centhaquin on systemic hemodynamics in hemorrhagic shock

Centhaquin produced a significant increase in cardiac output and augmentation of a hypertonic saline-induced increase in cardiac output of hemorrhaged rats. Supporting this finding is the observation that centhaquin produces a positive inotropic effect and increases ventricular contractions of isolated perfused rabbit heart along with an increase in the release of norepinephrine (Bhatnagar, Pande et al. 1985).

Hypotensive resuscitation has been suggested as an alternative to the current standard of care, i.e., produce a limited increase in blood pressure, which results in less use of resuscitation fluids and blood products. Indications are that this is a safe strategy for use in trauma patients (Morrison, Carrick et al. 2011). In an experimental study, a targeted blood pressure of 50-60 mmHg has been found to be ideal for treatment of hemorrhagic shock (Li, Zhu et al. 2011). Centhaquin-induced resuscitation produced an increase in blood pressure which was well below the baseline of 95 mmHg, i.e., a maximal increase in blood pressure following resuscitation with centhaquin was to about 65 to 75 mmHg. Blood pressure therefore increased in the range suggested to be ideal for hemorrhagic shock following resuscitation with centhaquin. The three doses of centhaquin used in these studies produced similar resuscitative effects, indicating a broad safety window. In a recent study, the amount of norepinephrine (NE) required to maintain MAP at 70 mmHg in normal saline (NS) or centhaquin (0.05 mg/kg) treated rats (volume equal to blood loss) was determined. Blood hematocrit decreased following hemorrhage and was similar in all the groups. Blood lactate was 4.10±1.02 mmol/L in NS compared to 1.65±0.23 mmol/L in centhaquin (P=0.041), 60 min following resuscitation. The amount of NE needed in each rat to maintain MAP at 70 mmHg was 175 µg in NS and 17.5 µg in centhaquin group during the first 60 min of resuscitation. Centhaquin was found to be a highly effective resuscitation agent decreasing the requirement of NE in hemorrhaged rats.

### Mechanism of action

The cardiovascular effects of centhaquin result from its action at α adrenergic receptors within the sympathetic nervous system, the heart, and the vasculature. There are two main types of adrenergic receptors α and β; which are subdivided into α₁ (α_{1A}, α_{1B} and α_{1D}) and α₂ (α_{2A}, α_{2B} and α_{2C}); and β₁, β₂, and β₃ adrenergic receptors (Bylund, Eikenberg et al. 1994). In a preliminary experiment, it was found that some of the pharmacological effects of centhaquin could be blocked by yohimbine which is a selective α₂ adrenergic receptor antagonist (Timmermans and Van Zwieten 1980; Sharma and Gulati 1995; Andurkar and Gulati 2011). The involvement of α₂ adrenergic receptors in the resuscitative effect of centhaquin therefore was investigated.

The following table shows the effect of centhaquin on arterial blood hematocrit, pH, blood gases, electrolytes, and lactate levels in the presence and absence of the specific α₂ adrenergic antagonists, yohimbine and atipamezole. Both yohimbine and atipamezole antagonized the resuscitative effect of centhaquin indicating that α₂ adrenergic receptors are involved in the resuscitative effect of centhaquin. Two different α₂ adrenergic receptor blockers, atipamezole which is a small molecule (Virtanen, Savola et al. 1989) and yohimbine which is a large complex alkaloid (Sharma and Gulati 1995; Kovacs and Hernadi 2003) were used, and both antagonists completely blocked the resuscitative effect of centhaquin, confirming that α₂ adrenergic receptors are involved in centhaquin resuscitation. It therefore can be concluded that drugs stimulating α₂ adrenergic receptors are useful in the treatment of shock. An increase in blood pressure that developed upon administration of centhaquin is therefore mostly driven by activation of vascular α₂ adrenergic receptors. Pharmacological data indicates that this activity results from an agonist-type response which is more likely due action on α_{2B} and α_{2C} adrenergic receptors (Vacher, Funes et al. 2010).

| **Parameters** | **Treatment** | **Baseline** | **Hemorrhage** | **Resuscitation 30 min** | **Resuscitation 60 min** | **Resuscitation 120 min** |
|---|---|---|---|---|---|---|
| Hematocrit | Vehicle + Centhaquin | 54.67 ± 2.03 | 37.67 ± 2.33 | 33.00 ± 1.53 | 32.67 ± 1.76 | 33.67 ± 2.03 |
| | Yohimbine + Centhaquin | 50.00 ± 1.26 | 35.60 ± 1.50 | 30.75 ± 1.38 | 31.50 ± 0.50 | |
| | Atipamezole + Centhaquin | 48.80 ± 1.66 | 36.20 ± 2.44 | 31.80 ± 1.96 | 29.00 ± 4.00 | |
| pH | Vehicle + Centhaquin | 7.34 ± 0.01 | 7.24 ± 0.02 | 7.29 ± 0.01 | 7.31 ± 0.01 | 7.28 ± 0.01 |
| | Yohimbine + Centhaquin | 7.34 ± 0.01 | 7.22 ± 0.02 | 7.19 ± 0.02* | 7.14 ± 0.05* | |
| | Atipamezole + Centhaquin | 7.36 ± 0.00 | 7.23 ± 0.03 | 7.25 ± 0.03 | 7.26 ± 0.01 | |
| pCO₂ | Vehicle + Centhaquin | 46.33 ± 1.67 | 27.33 ± 1.20 | 33.00 ± 0.58 | 33.33 ± 0.33 | 29.00 ± 1.15 |
| | Yohimbine + Centhaquin | 42.80 ± 1.32 | 26.60 ± 1.83 | 30.50 ± 0.96 | 35.00 ± 1.00 | |
| | Atipamezole + Centhaquin | 41.60 ± 1.91 | 26.60 ± 2.06 | 28.80 ± 1.98 | 30.50 ± 1.50 | |
| pO₂ | Vehicle + Centhaquin | 118.33 ± 6.77 | 124.00 ± 4.73 | 124.00 ± 4.51 | 121.67 ± 4.63 | 132.00 ± 6.69 |
| | Yohimbine + Centhaquin | 106.40 ± 2.11 | 127.80 ± 5.76 | 146.25 ± 5.66 | 117.00 ± 8.00 | |
| | Atipamezole + Centhaquin | 97.20 ± 1.02 | 113.60 ± 4.20 | 116.80 ± 2.35 | 115.50 ± 6.50 | |
| Na+ | Vehicle + Centhaquin | 134.33 ± 0.88 | 124.33 ± 1.86 | 130.00 ± 0.08 | 128.00 ± 1.00 | 130.33 ± 0.33 |
| | Yohimbine + Centhaquin | 130.60 ± 6.95 | 120.60 ± 4.59 | 125.75 ± 2.90 | 126.00 ± 2.00 | |
| | Atipamezole + Centhaquin | 141.40 ± 2.23 | 129.80 ± 1.85 | 133.60 ± 0.68 | 134.50 ± 0.50 | |
| K+ | Vehicle + Centhaquin | 2.73 ± 1.07 | 5.73 ± 0.30 | 4.73 ± 0.09 | 4.60 ± 0.00 | 5.17 ± 0.15 |
| | Yohimbine + Centhaquin | 3.58 ± 0.13 | 6.09 ± 0.42 | 5.33 ± 0.17 | 5.15 ± 0.15 | |
| | Atipamezole + Centhaquin | 3.46 ± 0.26 | 5.90 ± 0.25 | 4.94 ± 0.19 | 4.80 ± 0.20 | |
| Ca++ | Vehicle + Centhaquin | 1.15 ± 0.02 | 1.22 ± 0.00 | 1.17 ± 0.02 | 1.18 ± 0.03 | 1.18 ± 0.01 |
| | Yohimbine + Centhaquin | 1.10 ± 0.04 | 1.21 ± 0.05 | 1.18 ± 0.01 | 1.23 ± 0.01 | |
| | Atipamezole + Centhaquin | 1.09 ± 0.04 | 1.22 ± 0.02 | 1.18 ± 0.01 | 1.19 ± 0.04 | |
| Lactate | Vehicle + Centhaquin | 3.17 ± 0.24 | 10.10 ± 0.70 | 5.80 ± 0.32 | 4.53 ± 0.38 | 5.67 ± 0.22 |
| | Yohimbine + Centhaquin | 3.06 ± 0.22 | 10.34 ± 1.50 | 9.25 ± 0.33* | 8.40 ± 0.80* | |
| | Atipamezole + Centhaquin | 3.06 ± 0.23 | 10.76 ± 0.43 | 8.56 ± 0.68* | 7.75 ± 1.05* | |

Accordingly, α₂ adrenergic agents other than centhaquin are capable of improving cardiac output, organ perfusion, and tissue oxygenation, and are useful in the treatment of diseases and conditions in which these improvements provide a benefit.

Mice deficient in α_{2A} and α_{2C} adrenergic receptors developed normally and only additional deletion of α_{2B} gene led to placental defects (Philipp, Brede et al. 2002). Mice lacking all three α₂ subtypes did not survive beyond 11.5 days of embryonic development due to defect in the formation of fetal blood vessels in yolk sac and placenta (Philipp, Brede et al. 2002). Mice with selective inactivation of α_{2B} receptors are resistance to the development of salt-sensitive hypertension (Makaritsis, Handy et al. 1999; Makaritsis, Johns et al. 2000). In central vasomotor centers α_{2A} receptor subtypes are most abundant and produce a long lasting fall in blood pressure and increase in plasma norepinephrine (Tavares, Handy et al. 1996; Altman, Trendelenburg et al. 1999; Hein, Altman et al. 1999). Thus, both α_{2A} and α_{2B} adrenergic receptors regulate blood pressure, but the effects are opposite. Activation of central α_{2A} receptors decreases blood pressure by lowering sympathetic activity, and stimulation of central α_{2B} receptors contribute to the development of salt-mediated sympathetic increase in blood pressure (Gavras, Manolis et al. 2001). It is theorized that, at lower doses, centhaquin acts more on α_{2B} and α_{2C} adrenergic receptors to produce its resuscitative effect. However, at higher doses, the action of centhaquin on α_{2A} adrenergic receptors predominates, which leads to antihypertensive effects.

### Septic shock

Sepsis and septic shock continue to be a major challenge and contribute to significant mortality accounting for 9.3% of deaths (Angus, Linde-Zwirble et al. 2001). The number of severe sepsis hospitalizations per 100,000 persons increased from 143 in 2000 to 343 in 2007 (Kumar, Kumar et al. 2011). Severe sepsis is associated with development of septic shock which leads to insufficient oxygen delivery to organs and tissues resulting in cellular damage, multiple organ dysfunctions, and ultimately, death.

The objective measurement of systemic blood flow remains very challenging. Important indicators of circulatory failure are blood pressure, heart rate, urine output, capillary refill time, blood lactate concentration, central-peripheral temperature difference, pH, standard base excess, central venous oxygen saturation, and color (de Boode 2010). In septic shock, infusion of large volume of fluids for initial resuscitation is recommended (Brierley, Carcillo et al. 2009). The metabolic acidosis and base deficit due to the accumulation of lactic acid as a result of poor delivery of oxygen to the tissues serves as an indicator of the severity of the shock. Lactate levels are elevated in critically ill patients with sepsis and shock, and acetate levels appear to be an important indicator of outcome. Previous studies have documented elevated lactate levels in the rat model of endotoxic shock and hemorrhagic shock, and higher levels seem to correlate with greater short-term mortality (Gonzales, Chen et al. 2008; Sakai, Horinouchi et al. 2009).

Centhaquin is a cardiovascular active agent performing as a centrally acting sympatholytic agent. As disclosed above, centhaquin consistently reduced blood lactate levels from 2.42 ± 0.17 to 1.25 ± 0.55 mg/dL in normal rats. In previous experiments, centhaquin prolonged survival in hemorrhagic model of shock and was associated with reduction in blood lactate levels (Gulati, Lavhale et al. 2012). Both hemorrhagic shock and septic shock are associated with stress response with release of epinephrine and activation of inflammatory cascade and eventual multisystem organ failure (Cai, Deitch et al. 2010).

Because centhaquin is an effective resuscitation agent, centhaquin also is a useful agent in conditions of septic shock where it improves regional blood perfusion and reduces the hyper-activation of the sympathetic nervous system due to endotoxic shock.

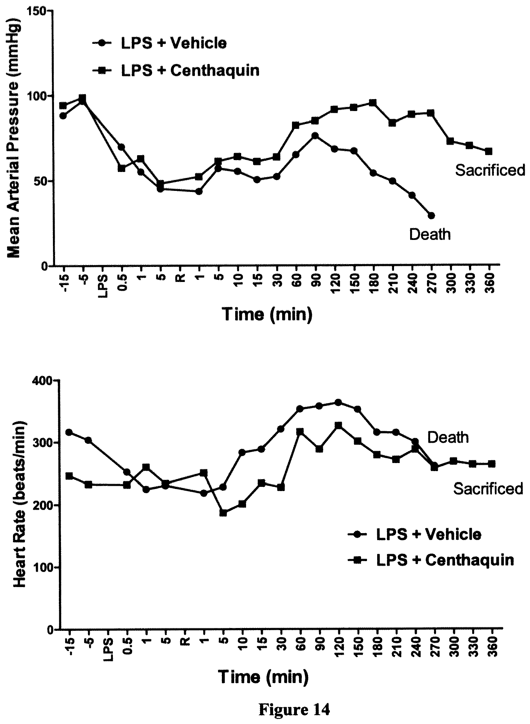

Figure 14 illustrates the efficiency of a low dose of centhaquin citrate in the treatment of septic shock. In the tests summarized in Figure 14, anaesthetized rats were immobilized on a surgical board and an incision was made above the femoral vein and artery. The vessels were cleaned and isolated. Heart rate and mean arterial pressure were measured by cannulating left femoral artery with pressure transducer SPR-320 (Millar Instruments), connected to a ML221 bridge amplifier (AD Instruments) through AEC-10C connector and the signals were acquired (1000 S⁻¹) using PowerLab 16/30. Left femoral veins were secured for drug administration using PE 50 tubing (Clay Adams, Parsipanny, NJ). Right femoral artery was cannulated for blood drawings using PE 50 tubing (Clay Adams, Parsipanny, NJ). After completion of the surgery, animals were maintained in a steady state for 30 minutes before administration of Lipopolysaccharide (LPS). Endotoxic shock was induced by intravenous injection of *E. coli* LPS O111:B4 (Sigma Chemical, St. Louis, MO) dissolved to a concentration of 10 mg in 0.1 ml of normal saline and a dose of 30 mg/kg was administered. Resuscitation (R) was performed either with vehicle (saline) or with centhaquin citrate (0.05 mg/kg). The rat treated with vehicle has lower blood pressure campared to the rat treated with centhaquin. Rat treated with vehicle died after 270 min, wherein the rat treated with centhaquin survived to 360 min and had to be sacrificed at the end of experiment. The dose of 0.05 mg/kg of centhaquin citrate in rat translates into an about 0.01 mg/kg dose in humans. These results clearly show the benefits of α₂ adrenergic agents, such as centhaquin and centhaquin citrate, in the treatment of septic shock.

### Dengue shock syndrome

An estimated 2.5 billion people worldwide are at risk of dengue infection, of which approximately 975 million people live in urban areas in tropical and sub-tropical countries in Southeast Asia, the Pacific, and the Americas (Guzman, Halstead et al. 2010). It is estimated that more than 50 million infections occur each year, including 500,000 hospitalizations for dengue haemorrhagic fever, mainly among children, of which about 5% are fatal (Guzman and Kouri 2002; Gubler 2004; Guzman and Isturiz 2010; Guzman, Halstead et al. 2010; San Martin, Brathwaite et al. 2010). The number of dengue cases reported to the World Health Organization (WHO) has increased dramatically in recent years. For the period 2000-2004, the annual average was 925,896 cases, almost double the figure of 479,848 cases that was reported for the period 1990-1999. In 2001, a record 69 countries reported dengue activity to WHO.

Dengue hemorrhagic fever and dengue shock syndrome are major causes of childhood morbidity and mortality in several tropical countries (Dung, Day et al. 1999). Dengue related shock results from capillary leakage of intravascular fluids, electrolytes, and small proteins into perivascular tissues, leading to pleural and pericardial effusions, decreasing blood pressure, low tissue perfusion and oliguria (Morens and Fauci 2008). In spite of normal hydration, the development of shock can be predicted by a gradually increasing hematocrit over a period of several hours. Fluid resuscitation to counter the massive plasma leakage is the main treatment.

In a randomized, blinded 230 patient study in children with dengue shock syndrome, a comparison between dextran, gelatin, Lactated Ringer's, and normal saline for initial resuscitation was carried out. All children survived and there was no clear advantage of using one fluid over the other (Premaratna, Liyanaarachchi et al. 2011). Pulse pressure was found to be the most significant factor determining clinical response of patients (Premaratna, Liyanaarachchi et al. 2011). In another study conducted on 383 patients, Lactated Ringer's was observed to be the treatment of choice in the initial stages in children with moderately severe dengue shock syndrome (Wills, Nguyen et al. 2005). In a retrospective chart review study, it was found that aggressive shock management tends to decrease the mortality rates in the severest forms of dengue shock syndrome (Ranjit, Kissoon et al. 2005). In a study conducted on ninety one children with serologically or PCR proven dengue virus infection, it was found that left ventricular ejection fraction and cardiac output were significantly lower in patients with dengue shock syndrome compared to patients of dengue hemorrhagic fever without shock (Khongphatthanayothin, Lertsapcharoen et al. 2007). Patients with shock had poor ventricular function and required more aggressive intravenous fluid resuscitation (Khongphatthanayothin, Lertsapcharoen et al. 2007).

It therefore can be concluded that issues addressing the choice of fluids, use of inotropes, and techniques of organ support are likely to yield benefits for the critically ill patients of dengue shock syndrome (Singhi, Kissoon et al. 2007).

As stated above, centhaquin consistently reduced blood lactate levels from 2.42 ± 0.17 to 1.25 ± 0.55 mg/dL in normal rats. It has been established that the odds of hyperlactatemia by logistic regression analysis are 4.6 (95% confidence interval 1.4-15; p < 0.05) times higher for a patient with abnormal peripheral perfusion (Lima, Jansen et al. 2009). Elevated lactate levels are associated with a worse prognosis for trauma patients. Inadequate oxygen delivery, disproportionate oxygen demand, and diminished oxygen use may lead to elevated lactate levels. Blood lactate monitoring is recommended in critical care settings and clearly has a place in the risk-stratification of critically ill patients (Jansen, van Bommel et al. 2009). Because centhaquin reduces blood lactate levels, centhaquin has a resuscitative effect in hemorrhagic shock.

Using a rodent model of fixed-pressure hemorrhage without tissue trauma with a base deficit of greater than -12 mEq/L (Gulati, Singh et al. 1995; Gulati, Sen et al. 1997; Gulati and Sen 1998) it was found that centhaquin enhanced the resuscitative effect of hypertonic saline. Centhaquin significantly decreased blood lactate, and increased mean arterial pressure (MAP), pulse pressure, and cardiac output (CO) compared to hypertonic saline alone. Centhaquin also decreased the mortality and increased the survival time of hemorrhaged rats compared to hypertonic saline. In another study, we found that blood lactate levels (mmol/L) increased from 7.22±0.67 at hemorrhage to 10.20±0.61 at 60 min following resuscitation with LR, while, blood lactate levels significantly decreased to 3.55±0.07 at 60 min following resuscitation with centhaquin. Centhaquin produced a 59% increase in MAP compared to a decrease of 29% by LR at 60 min following resuscitation.

This data shows that centhaquin and centhaquin citrate are effective in the treatment of dengue shock syndrome.

For each of the embodiments disclosed herein, pharmaceutical compositions containing the active agents of the present invention are suitable for administration to humans or other mammals. Typically, the pharmaceutical compositions are sterile, and contain no toxic, carcinogenic, or mutagenic compounds that would cause an adverse reaction when administered.

The method of the invention can be accomplished using the α₂ adrenergic agents as described above, or as a physiologically acceptable salt thereof. The α₂ adrenergic agents or salts can be administered as the neat compounds, or as a pharmaceutical composition containing either or both entities.

The α₂ adrenergic agents can be administered by any suitable route, for example by oral, buccal, inhalation, sublingual, rectal, vaginal, intracisternal through lumbar puncture, transurethral, nasal, percutaneous, i.e., transdermal, or parenteral (including intravenous, intramuscular, subcutaneous, and intracoronary) administration. Parenteral administration can be accomplished using a needle and syringe, or using a high pressure technique, like POWDERJECT™.

The pharmaceutical compositions include those wherein the α₂ adrenergic agents are administered in an effective amount to achieve their intended purpose. Determination of a therapeutically effective amount is well within the capability of those skilled in the art, especially in light of the detailed disclosure provided herein.

The exact formulation, route of administration, and dosage is determined by an individual physician in view of the patient's condition. Dosage amounts and intervals can be adjusted individually to provide levels of α₂ adrenergic agents that are sufficient to maintain therapeutic effects.

The amount of α₂ adrenergic agent administered is dependent on the subject being treated, on the subject's weight, the severity of the affliction, the manner of administration, and the judgment of the prescribing physician.

The α₂ adrenergic agents can be administered alone, or in admixture with a pharmaceutical carrier selected with regard to the intended route of administration and standard pharmaceutical practice. Pharmaceutical compositions for use in accordance with the present invention thus can be formulated in a conventional manner using one or more physiologically acceptable carriers comprising excipients and auxiliaries that facilitate processing of the α₂ adrenergic agents into preparations that can be used pharmaceutically.

These pharmaceutical compositions can be manufactured in a conventional manner, e.g., by conventional mixing, dissolving, granulating, dragee-making, emulsifying, suspending, encapsulating, entrapping, or lyophilizing processes. Proper formulation is dependent upon the route of administration chosen. When a therapeutically effective amount of the α₂ adrenergic agents are administered orally, the composition typically is in the form of a tablet, capsule, powder, solution, or elixir. When administered in tablet form, the composition can additionally contain a solid carrier, such as a gelatin or an adjuvant. The tablet, capsule, and powder contain about 5% to about 95% of an α₂ adrenergic agent, and preferably from about 25% to about 90% of an α₂ adrenergic agent. When administered in liquid form, the liquid form can contain any resuscitation fluid known in the art.

When a therapeutically effective amount of α₂ adrenergic agent is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, in addition to an α₂ adrenergic agent, an isotonic vehicle.

α₂ Adrenergic agents can be readily combined with pharmaceutically acceptable carriers well-known in the art. Such carriers enable the active agents to be formulated as tablets, pills, dragees, capsules, liquids, gels, syrups, slurries, emulsions, suspensions and the like, for oral ingestion by a patient to be treated. Pharmaceutical preparations for oral use can be obtained by adding the α₂ adrenergic agent with a solid excipient, optionally grinding the resulting mixture, and processing the mixture of granules, after adding suitable auxiliaries, if desired, to obtain tablets or dragee cores. Suitable excipients include, for example, fillers and cellulose preparations. If desired, disintegrating agents can be added.

The α₂ adrenergic agent can be formulated for parenteral administration by injection, e.g., by bolus injection or continuous infusion. Formulations for injection can be presented in unit dosage form, e.g., in ampules, vials, or in multidose containers, with an added preservative. The compositions can take such forms as suspensions, solutions, or emulsions in oily or aqueous vehicles, and can contain formulatory agents, such as suspending, emulsifying, stabilizing, and/or dispersing agents.

Pharmaceutical compositions for parenteral administration include aqueous solutions of the active agent in water-soluble form. Additionally, suspensions of the active agents can be prepared as appropriate oily injection suspensions. Suitable lipophilic solvents or vehicles include fatty oils or synthetic fatty acid esters. Aqueous injection suspensions can contain substances which increase the viscosity of the suspension. Optionally, the suspension also can contain suitable stabilizers or agents that increase the solubility of the compounds and allow for the preparation of highly concentrated solutions. Alternatively, a present composition can be in powder form for constitution with a suitable vehicle, e.g., sterile pyrogen-free water, before use.

As an additional aspect, the invention includes kits which comprise one or more compounds or compositions packaged in a manner which facilitates their use to practice methods of the invention. In a simplest embodiment, such a kit includes a compound or composition described herein as useful for practice of a method of the invention (i.e., centhaquin), packaged in a container such as a sealed bottle or vessel, with a label affixed to the container or included in the package that describes use of the compound or composition to practice the method of the invention. Preferably, the compound or composition is packaged in a unit dosage form. The kit may further include a device suitable for administering the composition according to a preferred route of administration.

### REFERENCES

Acosta et al. (1998). J Am Coll Surg 186(5): 528-33.
Alam et al. (2007). Surg Clin North Am 87(1): 55-72, vi.
Alam, H. B., K. Stanton, et al. (2004). Resuscitation 60(1): 91-9.
Altman, J. D., A. U. Trendelenburg, et al. (1999). Mol Pharmacol 56(1): 154-61.
Andurkar, S. V. and A. Gulati (2011). Pharmacology 88(5-6): 233-41.
Angus, D. C., W. T. Linde-Zwirble, et al. (2001). Crit Care Med 29(7): 1303-10.
Ayuste, E. C., H. Chen, et al. (2006). J Trauma 60(1): 52-63.
Bajpai, U. C., D. C. Gupta, et al. (2000). J. Molecular Structure 516: 15-21.
Balogh, Z., B. A. McKinley, et al. (2003). J Trauma 54(5): 848-59; discussion 859-61.
Barcroft, H. and O. G. Edholm (1945). J Physiol 104(2): 161-75.
Bellamy, R. F. (1984). Mil Med 149(2): 55-62.
Bhatnagar, M., M. Pande, et al. (1985). Arzneimittelforschung 35(4): 693-7.
Bickell, W. H., S. P. Bruttig, et al. (1991). Surgery 110(3): 529-36.
Bickell, W. H., S. P. Bruttig, et al. (1992). Ann Emerg Med 21(9): 1077-85.
Bickell, W. H., M. J. Wall, Jr., et al. (1994). N Engl J Med 331(17): 1105-9.
Brierley, J., J. A. Carcillo, et al. (2009). Crit Care Med 37(2): 666-88.
Bulger, E. M., S. May, et al. (2010). JAMA 304(13): 1455-64.
Bulger, E. M., S. May, et al. (2011). Ann Surg 253(3): 431-41.
Bylund, D. B., D. C. Eikenberg, et al. (1994). Pharmacol Rev 46(2): 121-36.
Cai, B., E. A. Deitch, et al. (2010). Mediators Inflamm 2010: 642462.
Carpy, A. and A. K. Saxena (1991). Acta Crystallographica C47: 227-229.
Cavun, S. and W. R. Millington (2001). Am J Physiol Regul Integr Comp Physiol 281(3): R747-52.
Chappell, D., M. Jacob, et al. (2008). Anesthesiology 109(4): 723-40.
de Boode, W. P. (2010). Early Hum Dev 86(3): 137-41.
Drabek, T., P. M. Kochanek, et al. (2011). Shock 35(1): 67-73.
Dries, D. J. (1996). Shock 6(5): 311-6.
Dubick, M. A., S. P. Bruttig, et al. (2006). Shock 25(4): 321-8.
Dung, N. M., N. P. Day, et al. (1999). Clin Infect Dis 29(4): 787-94.
Engstrom, M., U. Schott, et al. (2006). J Trauma 61(3): 624-8.
Ganster, F., M. Burban, et al. (2010). Crit Care 14(5): R165.
Gavras, L, A. J. Manolis, et al. (2001). J Hypertens 19(12): 2115-24.
Gill, F., T. Pelit, et al. (2011). Arzneimittelforschung 61(4): 229-33.
Gondre, M. and G. J. Christ (1998). J Pharmacol Exp Ther 286(2): 635-42.
Gonzales, E. R., H. Chen, et al. (2008). J Trauma 65(3): 554-65.
Gubler, D. J. (2004). Comp Immunol Microbiol Infect Dis 27(5): 319-30.
Gulati, A. (1992). Life Sci 50(2): 153-60.
Gulati, A., G. Hussain, et al. (1991). Drug Development Research 23(4): 307-323.
Gulati, A., M. S. Lavhale, et al. (2012). J Surg Res PMID: 22487389.
Gulati, A. and S. Rebello (1994). J Lab Clin Med 124(1): 125-33.
Gulati, A. and A. P. Sen (1998). Shock 9(1): 65-73.
Gulati, A., A. P. Sen, et al. (1997). Am J Physiol 273(2Pt 2): H827-36.
Gulati, A., R. Singh, et al. (1995). J Lab Clin Med 126(6): 559-70.
Gulati, A. and R. C. Srimal (1993). Eur J Pharmacol 230(3): 293-300.
Guzman, A. and R. E. Isturiz (2010). Int J Antimicrob Agents 36 Suppl 1: S40-2.
Guzman, M. G., S. B. Halstead, et al. (2010). Nat Rev Microbiol 8(12 Suppl): S7-16.
Guzman, M. G. and G. Kouri (2002). Lancet Infect Dis 2(1): 33-42.
Hardy, J. F., P. de Moerloose, et al. (2006). Can J Anaesth 53(6 Suppl): S40-58.
Hein, L., J. D. Altman, et al. (1999). Nature 402(6758): 181-4.
Henrion, D. and I. Laher (1993). Hypertension 22(1): 78-83.
Heslop, D. J., K. A. Keay, et al. (2002). Neuroscience 113(3): 555-67.
Hierholzer, C., B. Harbrecht, et al. (1998). J Exp Med 187(6): 917-28.
Ho, A. M., M. K. Karmakar, et al. (2001). J Trauma 51(1): 173-7.
Hsia, C. J. and L. Ma (2011). Artif Organs.
Jansen, T. C., J. van Bommel, et al. (2009). Crit Care Med 37(10): 2827-39.
Kauvar, D. S., R. Lefering, et al. (2006). J Trauma 60(6 Suppl): S3-11.
Khongphatthanayothin, A., P. Lertsapcharoen, et al. (2007). Pediatr Crit Care Med 8(6): 524-9.
Kovacs, P. and 1. Hernadi (2003). Neuroreport 14(6): 833-6.
Kowalenko, T., S. Stem, et al. (1992). J Trauma 33(3): 349-53; discussion 361-2.
Kumar, G., N. Kumar, et al. (2011). Chest 140(5): 1223-31.
Li, T., Y. Zhu, et al. (2011). Anesthesiology 114(1): 111-9.
Lima, A., T. C. Jansen, et al. (2009). Crit Care Med 37(3): 934-8.
Liu, L. M., J. A. Ward, et al. (2003). Shock 19(3): 208-14.
Makaritsis, K. P., D. E. Handy, et al. (1999). Hypertension 33(1): 14-7.
Makaritsis, K. P., C. Johns, et al. (2000). Hypertension 35(2): 609-13.
Malone, D. L., J. R. Hess, et al. (2006). J Trauma 60(6 Suppl): S91-6.
Martini, W. Z. and J. B. Holcomb (2007). Ann Surg 246(5): 831-5.
Morens, D. M. and A. S. Fauci (2008). JAMA 299(2): 214-6.
Morrison, C. A., M. M. Carrick, et al. (2011). J Trauma 70(3): 652-63.
Murthi, V. A., P. C. Jain, et al. (1976). U.S. Patent No. 3,983,121.
Murti, A., K. Bhandari, et al. (1989). Indian Journal of Chemistry Section B-Organic Chemistry Including Medicinal Chemistry 28(11): 934-942.
Murti, A., K. Bhandari, et al. (1989). Indian J Chem 28B: 934-942.
Pfeifer, R., P. Kobbe, et al. (2011). J Orthop Res 29(2): 270-4.
Philipp, M., M. E. Brede, et al. (2002). Nat Genet 31(3): 311-5.
Premaratna, R., E. Liyanaarachchi, et al. (2011). BMC Infect Dis 11: 52.
Ranjit, S., N. Kissoon, et al. (2005). Pediatr Crit Care Med 6(4): 412-9.
Rhee, P., D. Burris, et al. (1998). J Trauma 44(2): 313-9.
Rhee, P., E. Koustova, et al. (2003). J Trauma 54(5 Suppl): S52-62.
Sakai, H., H. Horinouchi, et al. (2009). Shock 31(5): 507-14.
San Martin, J. L., O. Brathwaite, et al. (2010). Am J Trop Med Hyg 82(1): 128-35.
Santry, H. P. and H. B. Alam (2010). Shock 33(3): 229-41.
Schadt, J. C. and J. Ludbrook (1991). Am J Physiol 260(2 Pt 2): H305-18.
Shackford, S. R., R. C. Mackersie, et al. (1993). Arch Surg 128(5): 571-5.
Sharma, A. C. and A. Gulati (1995). Crit Care Med 23(5): 874-84.
Singhi, S., N. Kissoon, et al. (2007). J Pediatr (Rio J) 83(2 Suppl): S22-35.
Srimal, R. C., K. Gulati, et al. (1990). Pharmacol Res 22(3): 319-29.
Srimal, R. C., D. F. J. Mason, et al. (1990). Asia Pac. J. Pharmacol. 5: 185-90.
Tabuchi, Y., M. Nakamaru, et al. (1989). Biochem Biophys Res Commun 159(3): 1304-8.
Tavares, A., D. E. Handy, et al. (1996). Hypertension 27(3 Pt 1): 449-55.
Timmermans, P. B. and P. A. Van Zwieten (1980). Eur J Pharmacol 63(2-3): 199-202.
Vacher, B., P. Funes, et al. (2010). J Med Chem 53(19): 6986-95.
Vincenzi, R., L. A. Cepeda, et al. (2009). Am J Surg 198(3): 407-14.
Virtanen, R., J. M. Savola, et al. (1989). Arch Int Pharmacodyn Ther 297: 190-204.
Watters, J. M., B. H. Tieu, et al. (2006). J Trauma 61(2): 300-8; discussion 308-9.
Watts, D. D., A. Trask, et al. (1998). J Trauma 44(5): 846-54.
Wills, B. A., M. D. Nguyen, et al. (2005). N Engl J Med 353(9): 877-89.
Wu, D., H. Dai, et al. (2009). Crit Care Med 37(6): 1994-9.
Zacharias, N., E. A. Sailhamer, et al. (2011). Resuscitation 82(1): 105-9.

## Claims

1. Centhaquin citrate for use in improving cardiac output, in an individual suffering from shock, wherein the centhaquin citrate is at a dose of 0.001 mg per kg to 0.05 mg per kg of weight of the individual.

2. The centhaquin citrate for use according to claim 1, wherein the centhaquin citrate is coadministered with a resuscitation fluid selected from the group consisting of a colloid solution, a crystalloid solution, blood, a blood component, a blood substitute, and mixtures thereof.

3. The centhaquin citrate for use according to claim 2, wherein the colloid solution or the crystalloid solution is selected from the group consisting of lactated Ringer's, saline, hypertonic saline, an albumin solution, a dextran solution, a gelatin solution, a hydroxyethyl starch solution, and a starch solution.

4. The centhaquin citrate for use according to claim 2, wherein the blood component is selected from the group consisting of plasma, red blood cells, white blood cells, platelets, clotting factors, proteins, and hormones.

5. The centhaquin citrate for use according to any of claims from 2 to 4, wherein the resuscitation fluid is administered in a volume amount of up to three times a volume fluid loss of the individual.

6. The centhaquin citrate for use according to claim 5, wherein the resuscitation fluid is administered in a volume amount of less than or up to a volume fluid loss of the individual.

7. The centhaquin citrate for use according to any of claims from 1 to 6, wherein the shock is hypovolemic shock, hemorrhagic shock, septic shock or dengue shock syndrome.

8. The centhaquin citrate for use according to any of claims from 1 to 7, which is a hydrate of centhaquin citrate.

9. The centhaquin citrate for use according to claim 8 wherein the hydrate is a monohydrate or dihydrate.

10. The centhaquin citrate for use according to claim 9 wherein the hydrate is dihydrate.

## Patentansprüche

1. Centhaquin-Citrat zur Verwendung bei der Verbesserung des Herzzeitvolumens in einem Individuum, das unter einem Schock leidet, wobei das Centhaquin-Citrat in einer Dosis von 0,001 mg pro kg bis 0,05 mg pro kg des Gewichts des Individuums vorliegt.

2. Centhaquin-Citrat zur Verwendung nach Anspruch 1, wobei das Centhaquin-Citrat mit einer Reanimationsflüssigkeit gemeinsam verabreicht wird, die ausgewählt ist aus der Gruppe bestehend aus einer Kolloidlösung, einer kristalloiden Lösung, Blut, einer Blutkomponente, einem Blutersatz und Mischungen davon.

3. Centhaquin-Citrat zur Verwendung nach Anspruch 2, wobei die Kolloidlösung oder die kristalloide Lösung ausgewählt ist aus der Gruppe bestehend aus Ringer-Lactatlösung, Kochsalzlösung, Hypertonischer Kochsalzlösung, einer Albuminlösung, einer Dextranlösung, einer Gelatinelösung, einer Hydroxyethylstärke-Lösung und einer Stärkelösung.

4. Centhaquin-Citrat zur Verwendung nach Anspruch 2, wobei die Blutkomponente ausgewählt ist aus der Gruppe bestehend aus Plasma, roten Blutkörperchen, weißen Blutkörperchen, Blutplättchen, Gerinnungsfaktoren, Proteinen und Hormonen.

5. Centhaquin-Citrat zur Verwendung nach einem der Ansprüche 2 bis 4, wobei die Reanimationsflüssigkeit in einer Volumenmenge von bis zu dreimal des Volumens des Flüssigkeitsverlusts des Individuums verabreicht wird.

6. Centhaquin-Citrat zur Verwendung nach Anspruch 5, wobei die Reanimationsflüssigkeit in einer Volumenmenge von weniger als oder bis zu einmal des Volumens des Flüssigkeitsverlusts des Individuums verabreicht wird.

7. Centhaquin-Citrat zur Verwendung nach einem der Ansprüche 1 bis 6, wobei der Schock hypovolämischer Schock, hämorrhagischer Schock, septischer Schock oder Dengue-Schock-Syndrom ist.

8. Centhaquin-Citrat zur Verwendung nach einem der Ansprüche 1 bis 7, das ein Hydrat von Centhaquin-Citrat ist.

9. Centhaquin-Citrat zur Verwendung nach Anspruch 8, wobei das Hydrat ein Monohydrat oder Dihydrat ist.

10. Centhaquin-Citrat zur Verwendung nach Anspruch 9, wobei das Hydrat ein Dihydrat ist.

## Revendications

1. Citrate de centhaquine pour son utilisation dans l'amélioration du débit cardiaque chez un individu se trouvant en état de choc, dans lequel le citrate de centhaquine est dosé à 0,001 mg par kg à 0,05 mg par kg de poids de l'individu.

2. Citrate de centhaquine pour son utilisation selon la revendication 1, dans lequel le citrate de centhaquine est administré conjointement avec un fluide de réanimation qui est sélectionné dans le groupe comprenant une solution colloïdale, une solution cristalloïde, le sang, un composant sanguin, un substitut sanguin ainsi que des mélanges de ceux-ci.

3. Citrate de centhaquine pour son utilisation selon la revendication 2, dans lequel la solution colloïdale ou la solution cristalloïde est sélectionnée dans le groupe comprenant une solution de Ringer lactée, une solution saline, une solution saline hypertonique, une solution d'albumine, une solution de dextrane, une solution de gélatine, une solution d'amidon hydroxyéthyle et une solution d'amidon.

4. Citrate de centhaquine pour son utilisation selon la revendication 2, dans lequel le composant sanguin est sélectionné dans le groupe comprenant le plasma, les globules rouges, les globules blancs, les plaquettes, les facteurs de coagulation, les protéines et les hormones.

5. Citrate de centhaquine pour son utilisation selon l'une quelconque des revendications 2 à 4, dans lequel le fluide de réanimation est administré en une quantité en volume pouvant aller jusqu'à trois fois une perte de fluide en volume de l'individu.

6. Citrate de centhaquine pour son utilisation selon la revendication 5, dans lequel dans lequel le fluide de réanimation est administré en une quantité en volume inférieure à ou jusqu'à une perte de fluide en volume de l'individu.

7. Citrate de centhaquine pour son utilisation selon l'une quelconque des revendications 1 à 6, dans lequel le choc est un choc hypovolémique, un choc hémorragique, un choc septique ou un syndrome de choc de dengue.

8. Citrate de centhaquine pour son utilisation selon l'une quelconque des revendications 1 à 7, qui est un hydrate de citrate de centhaquine.

9. Citrate de centhaquine pour son utilisation selon la revendication 8, dans lequel l'hydrate est un monohydrate ou un dihydrate.

10. Citrate de centhaquine pour son utilisation selon la revendication 9, dans lequel l'hydrate est un dihydrate.
